(19) Europäisches Patentamt
European Patent Office
Office européen des brevets

(11) EP 4 434 358 A1

(12) # EUROPEAN PATENT APPLICATION
published in accordance with Art. 153(4) EPC

(43) Date of publication:
**25.09.2024 Bulletin 2024/39**

(21) Application number: **22895594.4**

(22) Date of filing: **15.11.2022**

(51) International Patent Classification (IPC):
***A23L 33/10** (2016.01)* ***A23L 33/105** (2016.01)*
***A23L 33/12** (2016.01)* ***A61K 31/122** (2006.01)*
***A61K 31/192** (2006.01)* ***A61K 31/201** (2006.01)*
***A61P 1/14** (2006.01)* ***A61P 25/02** (2006.01)*
***A61P 43/00** (2006.01)* ***C07C 50/16** (2006.01)*
***C07C 66/02** (2006.01)*

(52) Cooperative Patent Classification (CPC):
**A23L 33/10; A23L 33/105; A23L 33/12; A61K 31/122; A61K 31/192; A61K 31/201; A61P 1/14; A61P 25/02; A61P 43/00; C07C 50/16; C07C 66/02**

(86) International application number:
**PCT/JP2022/042367**

(87) International publication number:
**WO 2023/090313 (25.05.2023 Gazette 2023/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **16.11.2021 JP 2021186575**
**24.02.2022 JP 2022026643**

(71) Applicant: **House Foods Group Inc.**
**Higashiosaka-shi, Osaka 577-8520 (JP)**

(72) Inventor: **IWASAKI Yusaku**
**Kyoto-shi, Kyoto 606-8522 (JP)**

(74) Representative: **Schön, Christoph**
**Dr. Schön, Neymeyr & Partner mbB**
**Bavariaring 26**
**80336 München (DE)**

(54) **COMPOSITION FOR INCREASING AMOUNT OF FOOD INGESTED AND/OR IMPROVING ANOREXIA, AND COMPOSITION FOR ACTIVATING TRANSIENT RECEPTOR POTENTIAL ANKYRIN 1**

(57) The purpose of the present invention is to newly discover a transient receptor potential ankyrin 1 (TRPA1) agonist that exhibits an action for increasing the amount of food ingested, to provide a novel agonist of TRPA1, or to provide a novel active ingredient that exhibits an action for increasing the amount of food ingested or an action for improving anorexia. A compound having a specific fatty acid or a specific anthraquinone structure has an effect for activating TRPA1 and can be used as an active ingredient for increasing the amount of food ingested and/or improving anorexia.



Processed by Luminess, 75001 PARIS (FR)

## Description

### Technical Field

The present invention relates to a composition for increasing an amount of food ingested and/or improving anorexia, and a composition for activating a transient receptor potential ankyrin 1 (TRPA1), and relates to a composition for increasing an amount of food ingested and/or improving anorexia, comprising a specific fatty acid or a compound having a specific anthraquinone structure. In addition, the present invention relates to a composition for activating TRPA1, comprising a compound having a specific anthraquinone structure.

### Background Art

TRPA1 is one of non-selective cation channels belonging to the super family of transient receptor potential (TRP) ion channels. TRPA1 is predominantly expressed mainly in the somatic sensory nerves (the somatic sensory nerves and the trigeminal nerves) and functions as a nociceptor involved in pain, pungency, and the like. In addition, besides the somatic sensory nerves, TRPA1 is often expressed also in the afferent vagal nerves which are involved in regulating intake of food.

As agonists that activate TRPA1, various compounds or plant extracts are known (Patent Literatures 1 to 9). Particularly, it is known that pungent components of spices or savory herbs include components that act as TRPA1 agonists, and such TRPA1 agonists exhibit an action of improving anorexia through an action of increasing an amount of food ingested (Patent Literature 10 and Non-Patent Literature 1). On the other hand, Patent Literature 11 states that a certain type of TRPA1 agonist exhibits an appetite suppressing action through an action of inducing secretion of cholecystokinin.

### Citation List

#### Patent Literatures

Patent Literature 1: Japanese Patent Application Publication No. 2018-177739
Patent Literature 2: Published Japanese Translation of PCT International Application No. 2017-518963
Patent Literature 3: Japanese Patent Application Publication No. 2017-096785
Patent Literature 4: Japanese Patent Application Publication No. 2014-210725
Patent Literature 5: Japanese Patent Application Publication No. 2014-169240
Patent Literature 6: Japanese Patent Application Publication No. 2014-076979
Patent Literature 7: Japanese Patent Application Publication No. 2014-024810
Patent Literature 8: Published Japanese Translation of PCT International Application No. 2011-506289
Patent Literature 9: International Publication No. WO2009/123080
Patent Literature 10: Japanese Patent Application Publication No. 2020-109065
Patent Literature 11: Japanese Patent Application Publication No. 2015-231953

#### Non-Patent Literature

Non-Patent Literature 1: Mishima Kaiun Memorial Foundation Research Report (2014), 51, 53 to 56

### Summary of Invention

#### Problems to be solved by the invention

From the conventional reports, it has not been clear whether an amount of food ingested is increased or lowered by TRPA1 agonists. In view of this, an object of the present invention is to newly find a TRPA1 agonist that exhibits an action of increasing an amount of food ingested, or to provide a novel agonist of TRPA1, or to provide a novel active ingredient that exhibits an action of increasing an amount of food ingested or an action of improving anorexia.

#### Means for solution of the problems

As a result of conducting earnest studies in order to solve the above problem, the present inventors found that

a certain type of fatty acid that has a TRPA1 activating action exhibits an action of increasing an amount of food ingested and thus completed one aspect of the present invention. In addition, the present inventors found that a compound having a specific anthraquinone structure exhibits a TRPA1 activating action and also exhibits an action of increasing an amount of food ingested and an action of improving anorexia, and completed a further aspect of the present invention. That is, the present invention provides a composition for increasing an amount of food ingested and/or improving anorexia as well as a composition for activating TRPA1.

- A composition for increasing an amount of food ingested and/or improving anorexia, the composition comprising

  (A) a fatty acid having 12 to 26 carbon atoms, 2 or more carbon-carbon double bonds, and 1 to 3 substituents selected from the group consisting of a hydroxyl group and a hydroperoxide group, or a pharmaceutically acceptable salt thereof, or a fatty acid which is an oxylipin having a TRPA1 activating action, or a pharmaceutically acceptable salt thereof, or
  (B) a compound represented by formula (1):

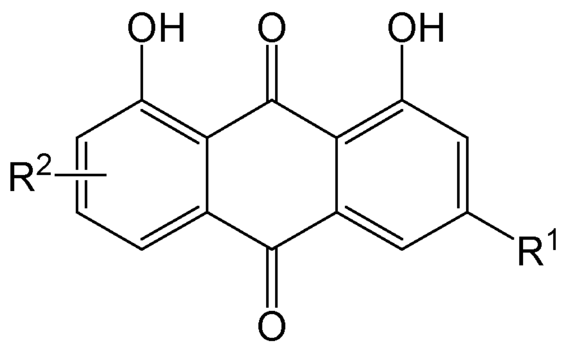

(1)

  wherein

    $R^1$ is -COOH, -OH, $-CH_2$-OH, or -O-$CH_3$, and
    $R^2$ is -H, $-CH_3$, or -OH, or a glycoside thereof, or a pharmaceutically acceptable salt thereof.

- A composition for activating TRPA1, comprising a compound represented by formula (1):

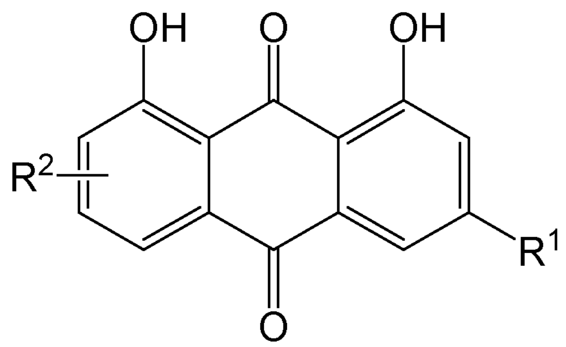

(1)

wherein

  $R^1$ is -COOH, -OH, $-CH_2$-OH, or -O-$CH_3$, and
  $R^2$ is -H, $-CH_3$, or -OH, or a glycoside thereof, or a pharmaceutically acceptable salt thereof.

As specific aspects of the present invention, for example, the following aspects are shown.

[1] A composition for increasing an amount of food ingested and/or improving anorexia, comprising a fatty acid having 12 to 26 carbon atoms, 2 or more carbon-carbon double bonds, and 1 to 3 substituents selected from the group consisting of a hydroxyl group and a hydroperoxide group, or a pharmaceutically acceptable salt thereof.
[2] A composition for increasing an amount of food ingested and/or improving anorexia, comprising a fatty acid which is an oxylipin having a TRPA1 activating action, or a pharmaceutically acceptable salt thereof.
[3] The composition according to the [1] or [2], wherein the fatty acid is represented by formula (1'):

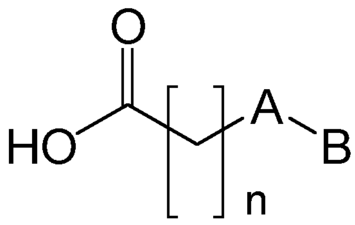

(1')

wherein

n is an integer of 3 to 9,
A is a saturated or unsaturated linear hydrocarbon group having 3 to 9 carbon atoms,
B is an unsaturated linear hydrocarbon group having 5 to 7 carbon atoms, and
A and B have a total of 2 or more carbon-carbon double bonds and a total of 1 to 3 substituents selected from the group consisting of a hydroxyl group and a hydroperoxide group.

[4] The composition according to the [3], wherein in the formula (1'), n is an integer of 6 to 8 and/or A is an unsaturated linear hydrocarbon group having 5 to 7 carbon atoms.
[5] The composition according to the [3] or [4], wherein in the formula (1'), B is

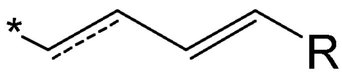

or

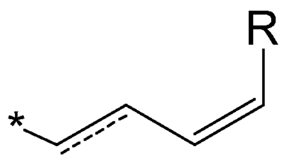

wherein asterisk represents a binding site with A, a solid double line and a dashed double line represent a single bond or a double bond, and R is an alkyl group having 1 to 3 carbon atoms.
[6] The composition according to any one of the [3] to [5], wherein in the formula (1'), A and B have a total of 2 to 5 carbon-carbon double bonds.
[7] The composition according to any one of the [3] to [6], wherein in the formula (1'),

n is an integer of 6 to 8,
A is an unsaturated linear hydrocarbon group having 5 to 7 carbon atoms, and
A and B have a total of 3 carbon-carbon double bonds and a total of one hydroxyl or hydroperoxide group.

[8] The composition according to any one of the [1] to [7], wherein a molecular formula of the fatty acid is $C_{18}H_{30}O_3$ or $C_{18}H_{32}O_3$.
[9] The composition according to any one of the [1] to [8], wherein the fatty acid has a structure represented by any of the following chemical formulas:

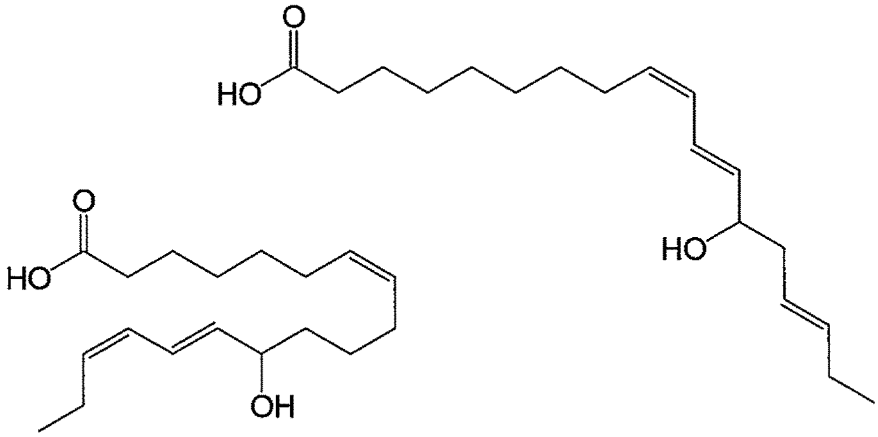

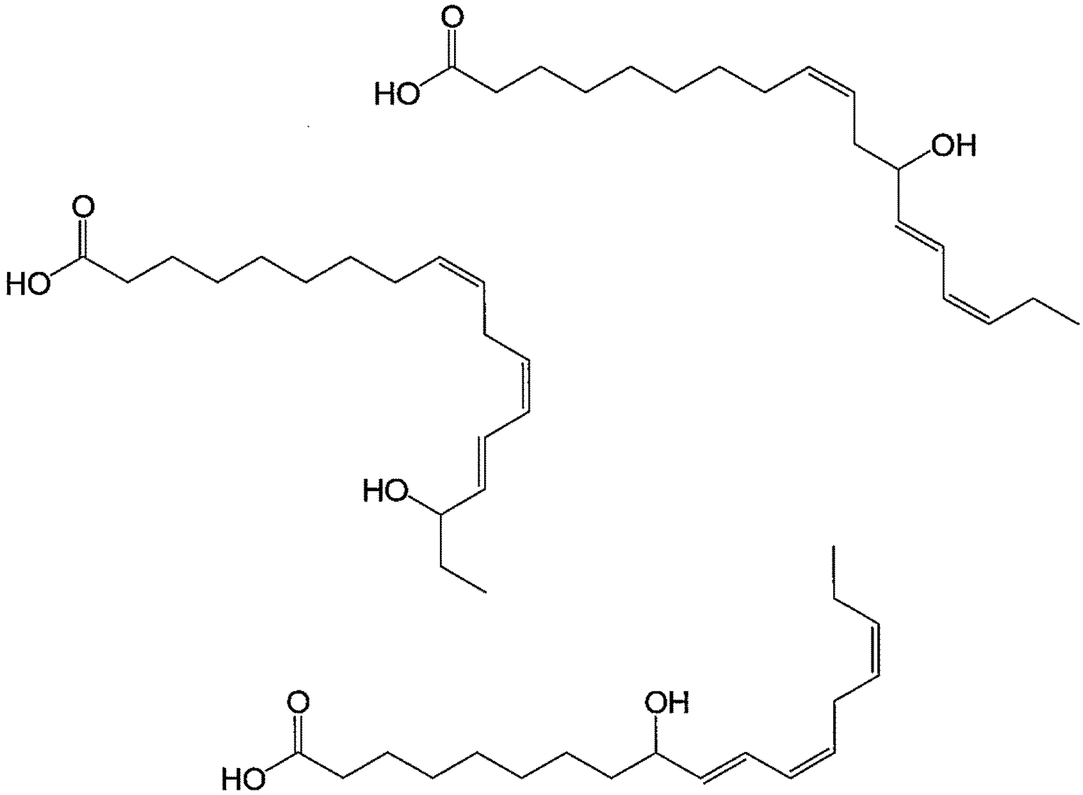

[10] The composition according to any one of the [1] to [9], that is a food composition or a pharmaceutical composition.
[11] A composition for activating TRPA1, comprising a compound represented by formula (1):

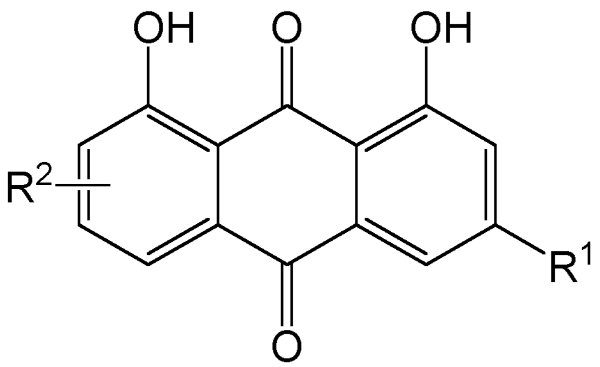

(1)

wherein

$R^1$ is -COOH, -OH, $-CH_2$-OH, or -O-$CH_3$, and
$R^2$ is -H, $-CH_3$, or -OH, or a glycoside thereof, or a pharmaceutically acceptable salt thereof.

[12] The composition according to the [11], wherein the compound is represented by formula (2):

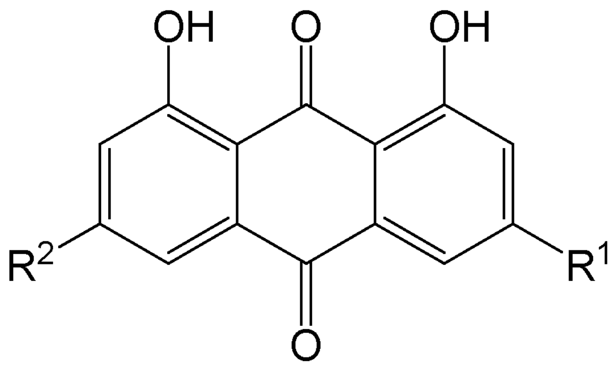

(2)

wherein

$R^1$ is -COOH, -OH, $-CH_2$-OH, or -O-$CH_3$, and
$R^2$ is -H, $-CH_3$, or -OH.

[13] The composition according to the [1] or [2], wherein the compound has any of the following structures:

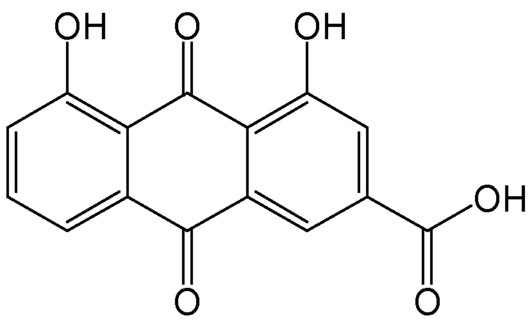

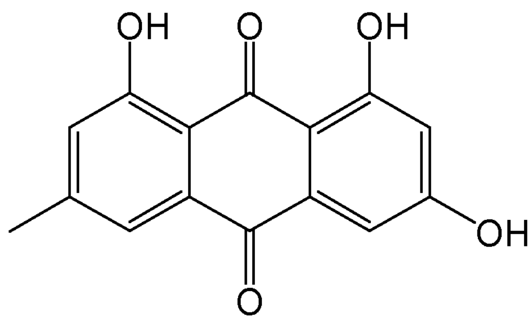

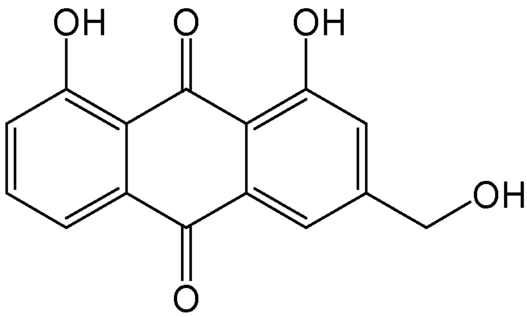

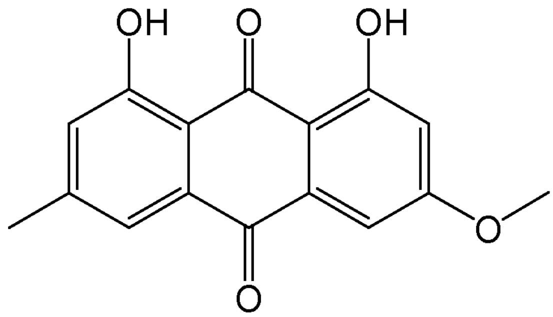

[14] The composition according to any one of the [11] to [13], that is a food composition, a pharmaceutical composition, or a pesticide composition.

[15] A composition for increasing an amount of food ingested and/or improving anorexia, comprising a compound represented by formula (1):

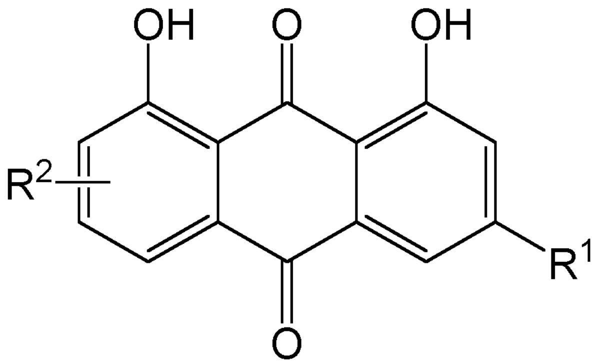

(1)

wherein

$R^1$ is -COOH, -OH, $-CH_2-OH$, or $-O-CH_3$, and
$R^2$ is -H, $-CH_3$, or -OH, or a glycoside thereof, or a pharmaceutically acceptable salt thereof.

[16] The composition according to the [15], wherein the compound is represented by formula (2):

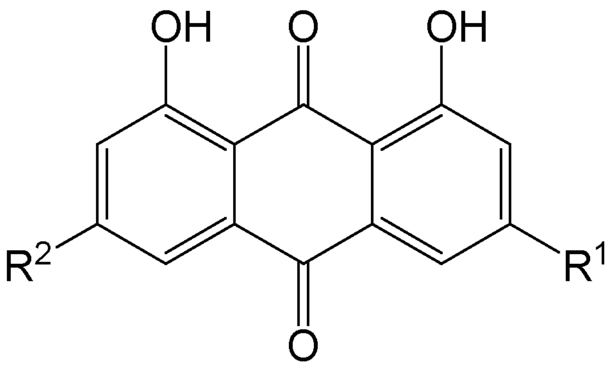

(2)

wherein

$R^1$ is -COOH, -OH, $-CH_2-OH$, or $-O-CH_3$, and

$R^2$ is -H, $-CH_3$, or -OH.

[17] The composition according to the [15] or [16], wherein the compound has any of the following structures:

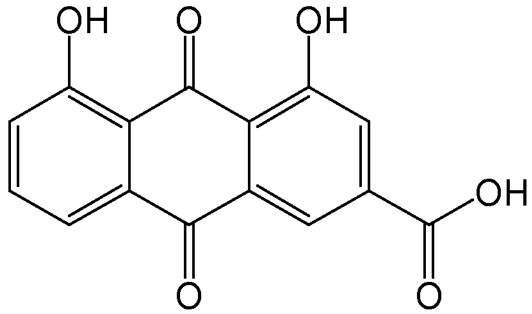

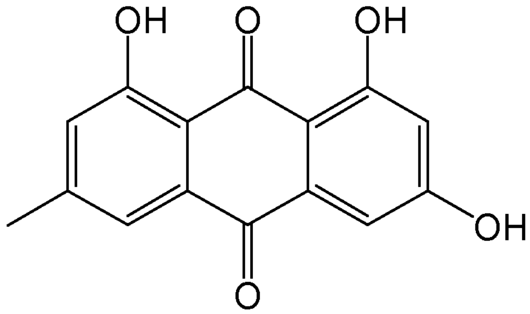

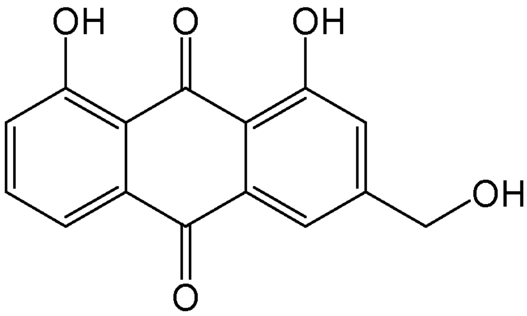

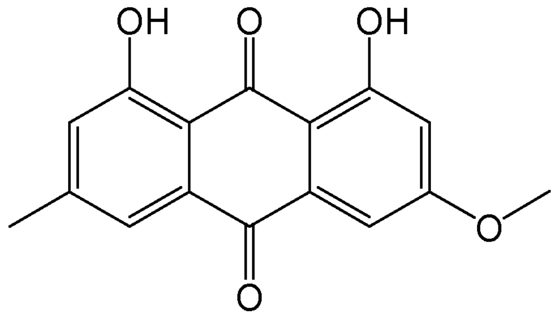

[18] The composition according to any one of the [15] to [17], that is a food composition or a pharmaceutical composition.

Advantageous Effects of Invention

According to the present invention, it is possible to stimulate appetite to increase an amount of food ingested by using an oxidized unsaturated fat such as oxylipins having a TRPA1 activating action. In addition, according to the present invention, it is possible to activate TRPA1 by using a compound represented by the formula (1) and stimulate appetite to increase an amount of food ingested. Hence, it becomes possible to improve anorexia by using an oxidized unsaturated fatty acid such as an oxylipin having a TRPA1 activating action or a compound represented by the formula (1).

Brief Description of Drawings

Fig. 1A shows a chromatogram of recycling preparative high performance liquid chromatography (HPLC).
Fig. 1B shows a chromatogram of recycling preparative HPLC.
Fig. 1C shows a chromatogram of recycling preparative HPLC.
Fig. 2 shows cumulative amounts of food ingested of mice with or without administration of a purified oxylipin.
Fig. 3 shows cumulative amounts of food ingested of mice with or without administration of rhein (300 μmol/kg body weight).
Fig. 4 shows cumulative amounts of food ingested of mice with or without administration of rhein (100 μmol/kg body weight).

Description of Embodiments

Hereinafter, the present invention will be described in further detail.
A composition of the present invention is a composition used for increasing an amount of food ingested and/or improving anorexia (a composition for increasing an amount of food ingested and/or improving anorexia), and in one aspect, comprises, as an active ingredient,

a fatty acid having 12 to 26 carbon atoms,
having 2 or more carbon-carbon double bonds, and
having 1 to 3 substituents selected from the group consisting of a hydroxyl group and a hydroperoxide group, or a pharmaceutically acceptable salt thereof, or
a fatty acid which is an oxylipin having a TRPA1 activating action, or a pharmaceutically acceptable salt thereof. A "pharmaceutically acceptable salt" described in the present Specification refers to a salt which exhibits an action of a free compound in vivo but is not harmful. The pharmaceutically acceptable salt of the fatty acid is not particularly limited but may be, for example, a metallic salt of an alkali metal, an alkaline earth metal, or the like. The pharmaceutically acceptable salt of the fatty acid may function by generating a free fatty acid in vivo.

An "oxylipin" described in the present Specification refers to an oxidized unsaturated fatty acid and has a hydroxyl group and/or a hydroperoxide group. The oxylipin may be extracted from a natural source or may be chemically synthesized. As a preparation method using extraction, a method normally used in the art can be employed without particular limitation, but the oxylipin may be extracted, for example, from various plants such as Gramineae plants including lemongrass and Solanaceae plants including tomato by using an organic solvent such as chloroform. More specifically, an oxylipin can be purified by

(1) extracting a dried powder of a plant by using chloroform,
(2) fractionating by using a medium-pressure preparative liquid chromatograph using a silica gel inject column and a silica gel purification column under the following conditions:

mobile phase: hexane/ethyl acetate
0 minutes (50%/50%)
16 minutes (50%/50%)
32 minutes (10%/90%)
48 minutes (10%/90%)
flow rate: about 40 mL/min, conducting a thin-layer chromatography (TLC) analysis using a silica gel plate and a developing solvent containing chloroform and methanol (chloroform/methanol=about 9/1), and collecting and combining fractions where a spot exhibiting an Rf value of 0.5 was detected,

(3) subjecting the collected fractions to preparative TLC using a silica gel plate and a developing solvent containing chloroform and methanol (chloroform/methanol=about 9/1), and scraping and collecting bands exhibiting an Rf value of 0.5 where absorption at a UV wavelength of 254 nm was detected,
(4) immersing the collected TLC bands in an extracting solvent containing chloroform and methanol (chloroform/methanol=about 8/2) to extract a component, and
(5) filtering the extract and concentrating the filtered extract to dryness.

Since the oxylipin thus purified can be a mixture of multiple oxylipin compounds, these may be isolated and used. For example, compounds contained in the oxylipin thus purified by the above method may be isolated by subjecting the oxylipin to recycling preparative HPLC under the following conditions:

preparative column: column in which dihydroxypropyl groups were chemically bonded to silica gel
mobile phase: hexane/ethanol=9/1
flow rate: about 10 mL/min
detection wavelength: 230 nm, to separate peaks which are separated every time the number of cycles increases, and if necessary, subjecting fractions containing the separated peaks to further recycling preparative HPLC under the same conditions or while changing the conditions as appropriate (for example, such as changing the mobile phase to chloroform/methanol=about 95/5 to about 99/1), and separating individually detected peaks.

In a certain dihydroxy propyl aspect, the fatty acid is represented by formula (1):

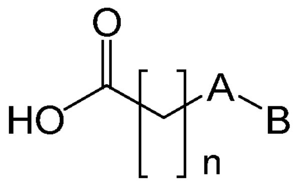

(1).

In the formula (1), n is an integer of 3 to 9 and preferably an integer of 6 to 8.

A is a saturated or unsaturated linear hydrocarbon group and has 3 to 9 and preferably 5 to 7 carbon atoms. In a certain aspect, A may be an unsaturated linear hydrocarbon group having 5 to 7 carbon atoms.

B is an unsaturated linear hydrocarbon group having 5 to 7 carbon atoms. In a certain aspect, B is

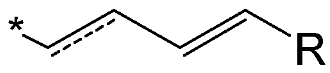

or

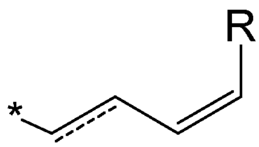

wherein asterisk represent a binding site with A, a solid double line and a dashed double line represent a single bond or a double bond, and R may be an alkyl group having 1 to 3 carbon atoms. In addition, in a certain aspect, B is

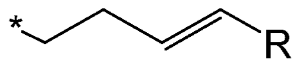

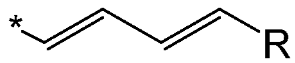

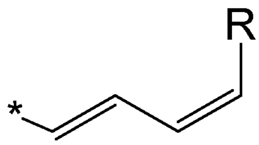

or

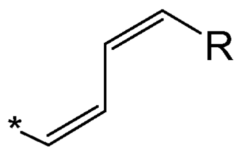

wherein asterisk represents a binding site with A, and R may be an alkyl group having 1 to 3 carbon atoms.

The unsaturated linear hydrocarbon groups of A and B contain carbon-carbon double bonds, and in the formula (1), A and B have a total of 2 or more and preferably 2 to 5 carbon-carbon double bonds and a total of 1 to 3 substituents selected from the group consisting of a hydroxyl group and a hydroperoxide group.

In a certain aspect, in the formula (1),

n is an integer of 6 to 8,
A is an unsaturated linear hydrocarbon group having 5 to 7 carbon atoms, and

A and B have a total of 3 carbon-carbon double bonds and a total of one hydroxyl or hydroperoxide group. In addition, in a certain aspect, a molecular formula of the fatty acid is $C_{18}H_{30}O_3$ or $C_{18}H_{32}O_3$ and preferably $C_{18}H_{30}O_3$.

In a certain aspect, the fatty acid has a structure represented by any of the following chemical formulas:

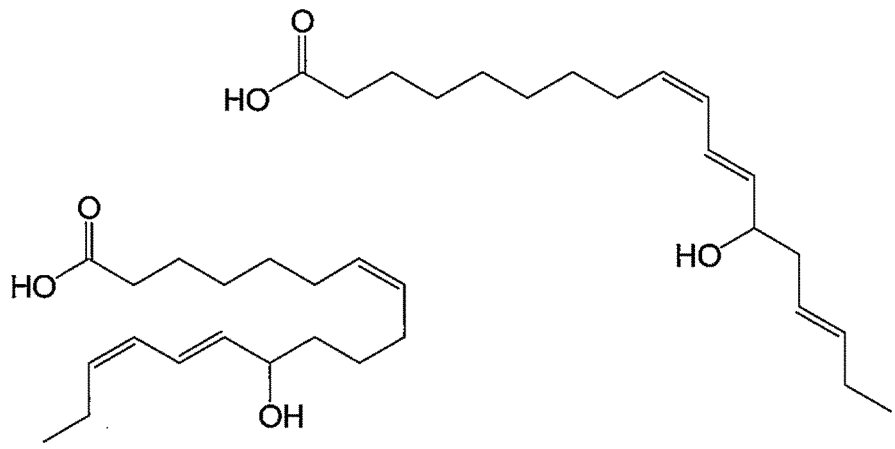

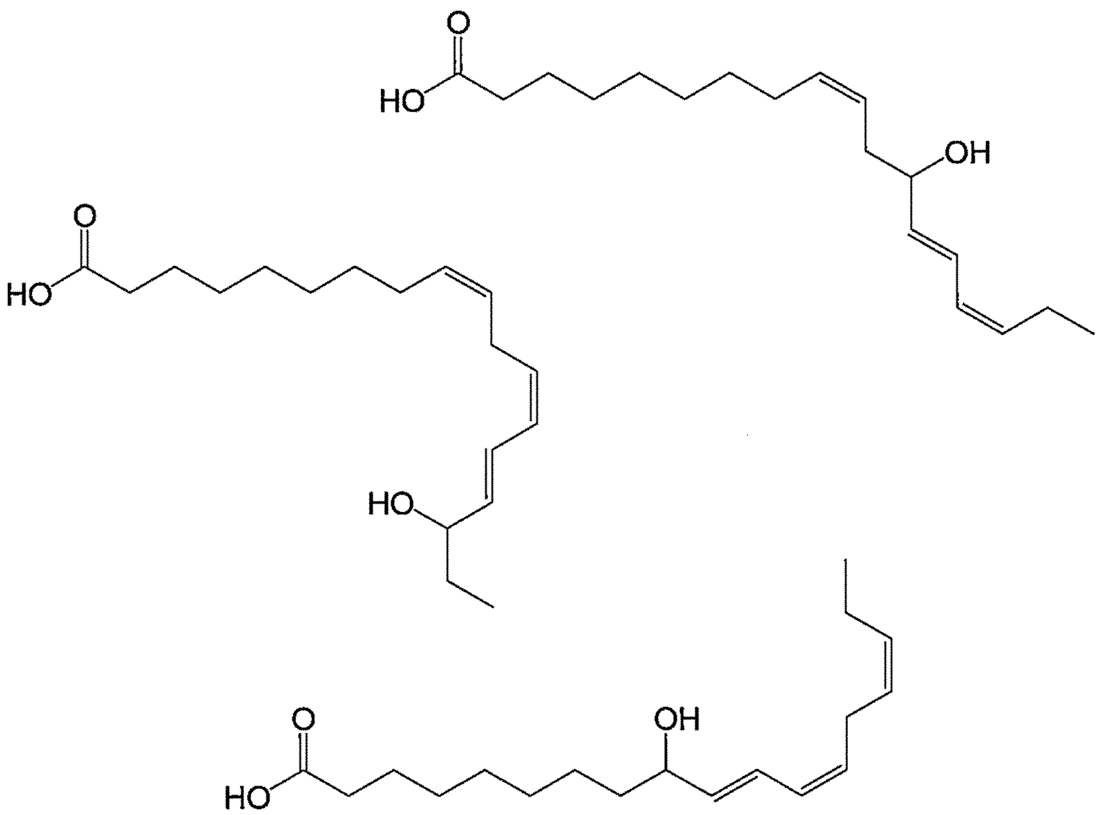

In another aspect of the composition for increasing an amount of food ingested and/or improving anorexia of the present invention, the composition comprises, as an active ingredient, a compound represented by formula (1):

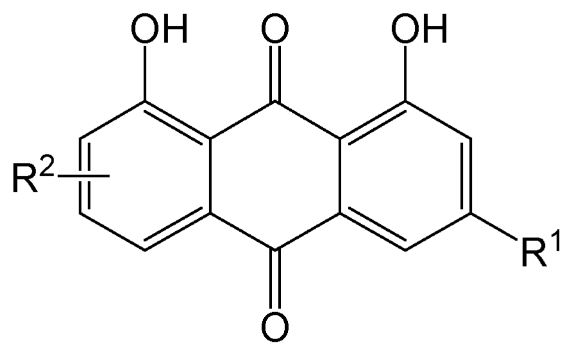

(1)

wherein

$R^1$ is -COOH, -OH, -$CH_2$-OH, or -O-$CH_3$, and

$R^2$ is -H, -$CH_3$, or -OH, or a glycoside thereof, or a pharmaceutically acceptable salt thereof. The compound, or a glycoside thereof, or a pharmaceutically acceptable salt thereof may be synthesized by a conventional method or may be extracted from a plant by a conventional method. In some cases, the composition of the present invention may comprise the compound, or a glycoside thereof, or a pharmaceutically acceptable salt thereof, in a form of a plant extract, a crude drug, or the like. As specifically described in Examples, which will be described later, the compound, which has a 1,8-dihydroxyanthraquinone structure including a substituent containing an oxygen atom at the 3 position, has the action of activating TRPA1, and it is considered that the composition functions through this action.

A "glycoside" described in the present Specification is a compound in which one or more monosaccharides are glycosidically bonded to the compound. The monosaccharide is not particularly limited but may contain, for example, glucose, maltose, galactose, or the like. In a certain aspect, the glycoside may contain an 8-O-glucoside compound of the compound.

The pharmaceutically acceptable salt of the compound is not particularly limited but may contain, for example, an alkali metal salt (a sodium salt, a potassium salt, or the like), an alkaline earth metal salt (a calcium salt or the like), a magnesium salt, or the like.

A content of the compound, or a glycoside thereof, or a pharmaceutically acceptable salt thereof in the composition of the present invention is not particularly limited as long as the amount of food ingested can be increased or anorexia can be improved, but may be, for example, about 0.069% by mass to about 0.853% by mass, and preferably about 0.853% by mass, relative to the total mass of the composition in terms of a free form of the compound.

In a certain aspect, the compound is represented by formula (2):

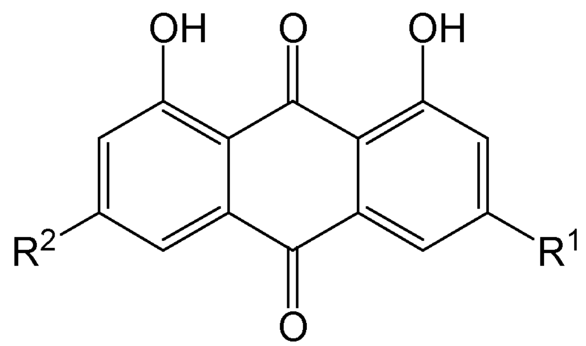

(2)

wherein

$R^1$ is -COOH, -OH, -$CH_2$-OH, or -O-$CH_3$, and

$R^2$ is -H, -$CH_3$, or -OH. More specifically, the compound may have any of the following structures:

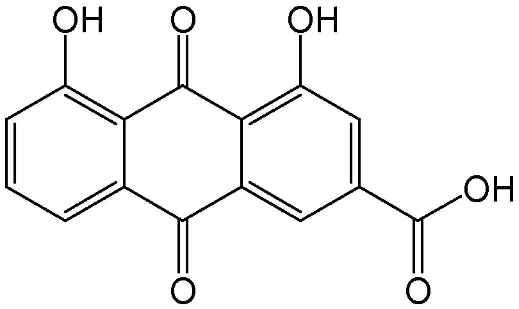

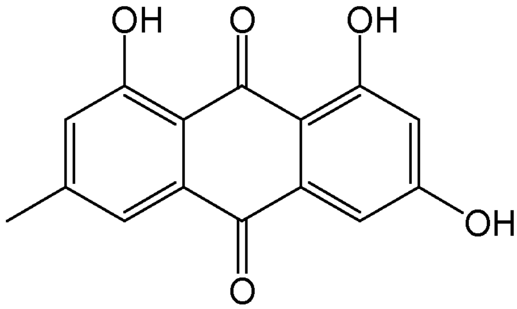

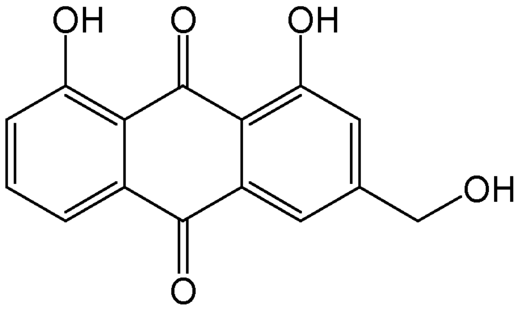

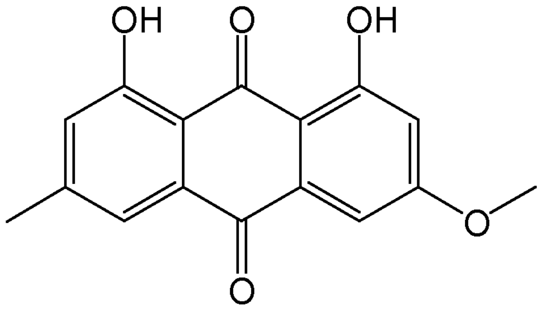

. That is, the compound may contain at least one selected from the group consisting of rhein, emodin, physcion, and aloe-emodin.

Without bound to a particular theory, since it is known that the TRPA1 agonist conveys peripheral information to the brain via sensory nerves including the afferent vagal nerve to increase the amount of food ingested of the administration target (Non Patent Literature 1), it is considered that the fatty acid specified by the present invention or the compound represented by the formula (1) also increases the amount of food ingested or improve anorexia by a similar mechanism.

In a certain aspect, the composition for increasing an amount of food ingested and/or improving anorexia of the present invention is a food composition or a pharmaceutical composition. This composition can be used in order to increase the amount of food ingested of a subject, stimulate the appetite of a subject, or improve anorexia of a subject. Hence, the composition may be provided with, for example, an indication as follows:

- For people who have low appetites
- For people who want to eat as much as they want
- For people who want to bulk up the bodies
- For people who want to maintain the amounts of diets which tend to decrease
- For people who want to maintain the motivation for diets.

The subjects to which the composition for increasing an amount of food ingested and/or improving anorexia of the present invention is applied are not particularly limited but may be, for example, humans (specifically, humans in convalescence after illness, humans losing appetite due to aging, children having difficulty in growths, athletes, humans having low stress resistances, and the like) or other mammals (pets such as dogs and cats and livestock such as pigs and cattle).

The composition for increasing an amount of food ingested and/or improving anorexia of the present invention may further comprise any inactive ingredient normally used in the art, for example, an additive such as a pharmaceutically or food acceptable solvent, a buffering agent, a sweetener, an acidulant, a flavor, an antifoam, and an antioxidant as long as the object of the present invention is not impaired. In addition, the composition may further comprise an additional active ingredient normally used in the art as long as the object of the present invention is not impaired. Such additional active ingredient may be an ingredient having an action of improving an amount of food ingested or an action of improving anorexia, or may be an ingredient having another action.

In another aspect, the present invention also relates to a composition for activating TRPA1, and comprises, as an active ingredient, a compound represented by formula (1):

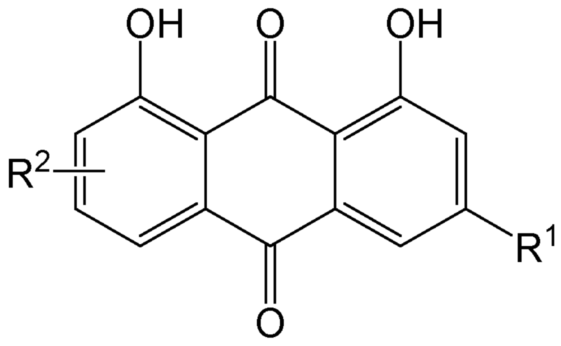

(1)

wherein

$R^1$ is -COOH, -OH, -$CH_2$-OH, or -O-$CH_3$, and
$R^2$ is -H, -$CH_3$, or -OH, or a glycoside thereof, or a pharmaceutically acceptable salt thereof. The glycoside and the pharmaceutically acceptable salt are as described in the present Specification. In addition, in the same manner as described above, the compound, or a glycoside thereof, or a pharmaceutically acceptable salt thereof may be synthesized by a conventional method or may be extracted from a plant by a conventional method, or may be blended in a form of a plant extract, a crude drug, or the like. In some cases, the composition of the present invention may comprise the compound, or a glycoside thereof, or a pharmaceutically acceptable salt thereof in a form of a plant extract, a crude drug, or the like.

A content of the compound, or a glycoside thereof, or a pharmaceutically acceptable salt thereof in the composition of the present invention is not particularly limited as long as TRPA1 can be activated, but may be, for example, about 0.069% by mass to about 0.853% by mass, and preferably about 0.853% by mass, relative to the total mass of the composition in terms of a free form of the compound.
In a certain aspect, the compound is represented by formula (2):

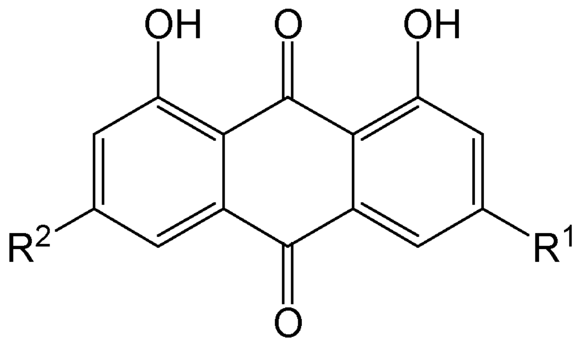

(2)

wherein

$R^1$ is -COOH, -OH, -$CH_2$-OH, or -O-$CH_3$, and
$R^2$ is -H, -$CH_3$, or -OH. More specifically, the compound may have any of the following structures:

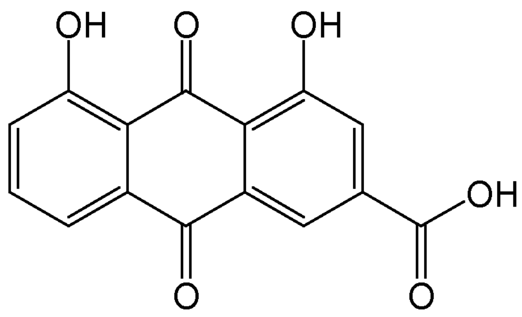

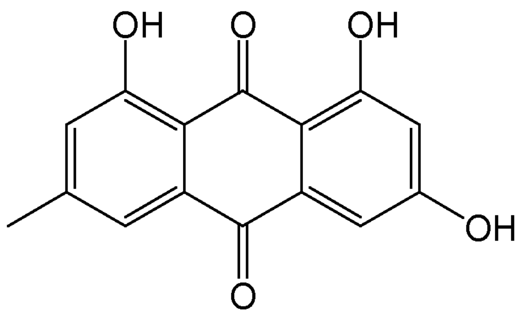

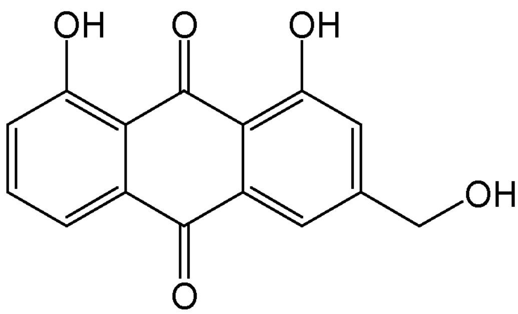

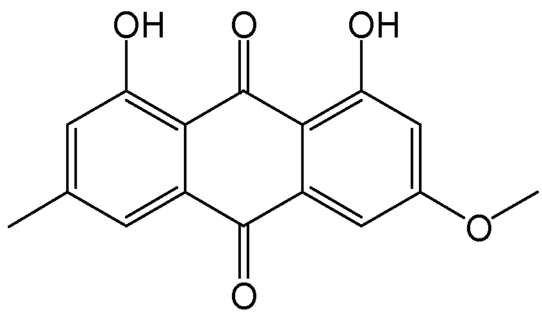

. That is, the compound may contain at least one selected from the group consisting of rhein, emodin, physcion, and aloe-emodin.

In addition, as another aspect, the present Specification also discloses an invention of a composition for activating TRPA1, comprising, as an active ingredient, an oxylipin derived from lemongrass or a pharmaceutically acceptable salt thereof. The pharmaceutically acceptable salt is not particularly limited but may be, for example, a metallic salt of an alkali metal, an alkaline earth metal, or the like. The pharmaceutically acceptable salt may function by generating a free form compound in vivo.

In a certain aspect, the oxylipin is a compound represented by formula (2):

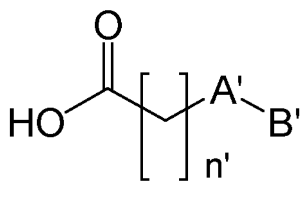

(2)

. That is, the present Specification also discloses an invention of a composition for activating TRPA1, comprising a compound represented by the formula (2) or a pharmaceutically acceptable salt thereof.

In the formula (2), n' is an integer of 3 to 9 and preferably an integer of 6 to 8.

A' is a saturated or unsaturated linear hydrocarbon group, and has 3 to 9 and preferably 5 to 7 carbon atoms. In a certain aspect, A' may be an unsaturated linear hydrocarbon group having 5 to 7 carbon atoms.

B' is

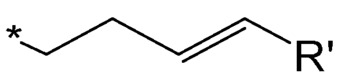

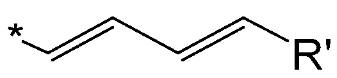

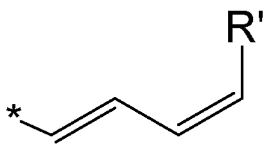

or

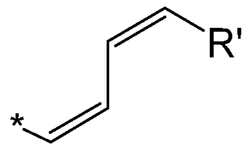

wherein asterisk represents a binding site with A', and R' is an alkyl group having 1 to 3 carbon atoms.

In the formula (2), A' and B' have a total of 2 or more and preferably 2 to 5 carbon-carbon double bonds and a total of 1 to 3 substituents selected from the group consisting of a hydroxyl group and a hydroperoxide group.

In a certain aspect, in the formula (2),

n' is an integer of 6 to 8,
A' is an unsaturated linear hydrocarbon group having 5 to 7 carbon atoms, and
A' and B' have a total of 3 carbon-carbon double bonds and a total of one hydroxyl or hydroperoxide group. In addition, in a certain aspect, a molecular formula of the compound is $C_{18}H_{30}O_3$ or $C_{18}H_{32}O_3$ and preferably $C_{18}H_{30}O_3$.

In a certain aspect, the compound has a structure represented by any of the following chemical formulas:

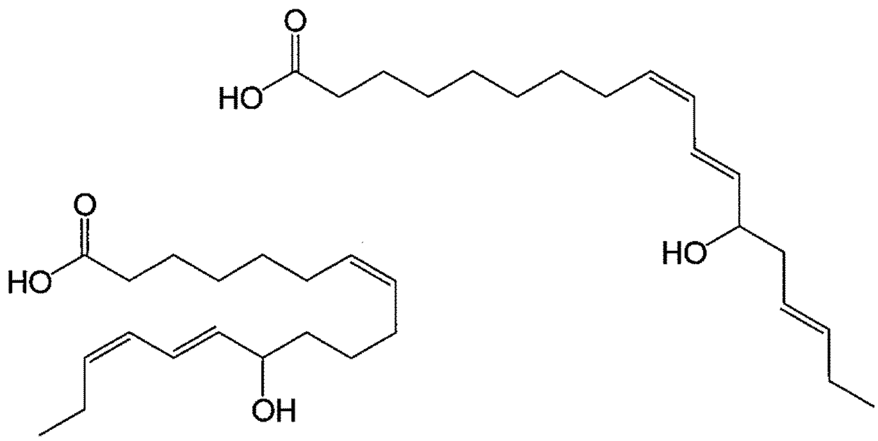

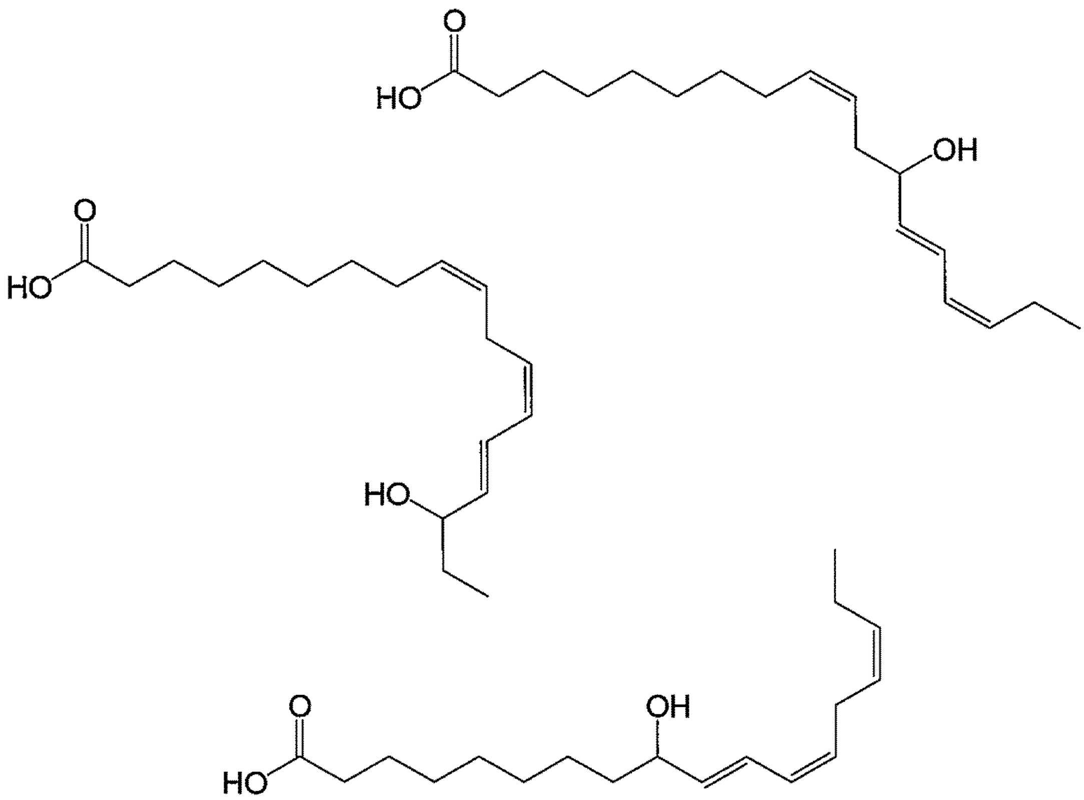

In a certain aspect, the composition for activating TRPA1 is a food composition, a pharmaceutical composition, or a pesticide composition. This composition can be used in order to add pungency to a target food, increase the amount of food ingested of a subject, stimulate the appetite of a subject, improve anorexia, respiratory failure, intestinal obstruction, or constipation of a subject, increase the energy metabolism of a subject to prevent or improve obesity, or repel pest insects, or other purposes, through the TRPA1 activating action.

The composition for activating TRPA1 may further comprise any inactive ingredient normally used in the art, for example, an additive such as a pharmaceutically or food acceptable solvent, a buffering agent, a sweetener, an acidulant, a flavor, an antifoam, and an antioxidant as long as the object of the invention is not impaired. In addition, the composition for activating TRPA1 may further comprise an additional active ingredient normally used in the art as long as the object of the invention is not impaired, and such additional active ingredient may be an ingredient having the TRPA1 activating action or may be an ingredient having another action.

Hereinafter, the present invention will be specifically described by using Examples; however, the scope of the present invention is not limited to these Examples. Examples

1. Extraction, purification, and isolation of oxylipin

To 2 L of chloroform put in an Erlenmeyer flask, about 100 g of a dried powder of lemongrass was added, followed by extracting for 12 hours or more under room temperature condition while stirring using a stirrer. A supernatant was collected through suction filtration and concentrated to dryness by using a rotary evaporator.

2 to 5 g of the obtained dried concentrate was dissolved in 20 mL of an initial solvent (hexane/ethyl acetate=50%/50%), and the resultant was introduced into a medium-pressure preparative liquid chromatograph and fractionated in 15 mL each under the following conditions.

Chromatograph: EPCLC-W-Prep 2XY (Yamazen Corporation)
Silica gel inject column: W830
Purification column: Si-40D (silica gel column)
Mobile phase: hexane/ethyl acetate
0 minutes (50%/50%)
16 minutes (50%/50%)
32 minutes (10%/90%)
48 minutes (10%/90%)

flow rate: 40 mL/min

TLC analysis was conducted on each fraction under the following conditions and fractions were collected and combined for a retention time from 28 minutes to 40 minutes where a spot exhibiting an Rf value of 0.5 was detected to obtain an oxylipin roughly purified fraction.

Silica gel plate: TLC glass plate silica gel 60 $F_{254}$ (Merck Millipore, product number 105715)

Developing solvent: chloroform/methanol=9/1

Coloring reagent: phosphomolybdic acid coloring reagent (ethanol solution containing 10% of sodium phosphomolybdate n-hydrate)

The oxylipin roughly purified fraction was subjected to preparative TLC under the following conditions and bands exhibiting an Rf value of 0.5 where absorption at a UV wavelength of 254 nm was detected were scraped and collected.

Silica gel plate: PLC glass plate silica gel 60 $F_{254}$, 1 mm (Merck Millipore, model number 113895)

Developing solvent: chloroform/methanol=9/1

The collected TLC bands were immersed in an extracting solvent (chloroform/methanol=8/2) in a volume ratio of 5 times or more, followed by stirring using a stirrer for 30 minutes or more to extract a component. The extract was filtered by using a paper filter and concentrated to dryness by using a rotary evaporator to obtain a purified oxylipin.

The purified oxylipin was subjected to recycling preparative HPLC under the following conditions to separate three peaks detected individually. The separated fractions were named R1, R2, and R3, respectively, in ascending order of retention time. Specifically, as shown in Fig. 1A, first a peak (R3) having the longest retention time was separated among three peaks detected at the 4th cycle, thereafter a peak (R2) having a longer retention time among peaks which were clearly separated was separated at the 8th cycle, and lastly the remaining peak (R1) was separated at the 10th cycle.

Recycling preparative device: device of Japan Analytical Industry Co., Ltd. (model number: LC-9110)

Preparative column: column in which dihydroxypropyl groups were chemically bonded to silica gel (GL Sciences Inc., product name Inertsil(R) Diol, particle size 5 μm, inner diameter 14 mm, length 250 mm, model number 5020-79054)

Mobile phase: hexane/ethanol=9/1

Flow rate: 9.999 mL/min

Detection wavelength: 230 nm

The R1 fraction and the R2 fraction were each individually subjected to recycling preparative HPLC under the following conditions, and peaks individually detected were each separated. For the R2 fraction, as shown in Fig. 1B, two peaks which separated as the number of cycles increased were separated, and a compound R2-1 having a short retention time and a compound R2-2 having a long retention time were obtained. For the R1 fraction, a compound R1-1 having a short retention time was obtained when the number of cycles was small, and a compound R1-2 having a long retention time was obtained after the number of cycles sufficiently increased. The R3 fraction was subjected to recycling preparative HPLC under the following conditions to increase the purity, so that a compound R3 was obtained. Note that the content ratio of the compounds R1-2, R2-1, R2-2, and R3 in the purified oxylipin was roughly 3:2:2:2.

Recycling preparative device: device of Japan Analytical Industry Co., Ltd. (model number: LC-9110)

Preparative column: column in which dihydroxypropyl groups were chemically bonded to silica gel (GL Sciences Inc., product name Inertsil(R) Diol, particle size 5 μm, inner diameter 14 mm, length 250 mm, model number 5020-79054)

Mobile phase: chloroform/methanol=98/2

Flow rate: 9.999 mL/min

Detection wavelength: 230 nm

The molecular formulas and molecular weights of the obtained compounds were determined as described in Table 1 in accordance with Orbitrap LC-MS analysis.

Table 1: Molecular formulas and molecular weights of compounds

| Compound | Molecular formula | Molecular weight |
|---|---|---|
| R1-1 | $C_{18}H_{32}O_3$ | 296 |
| R1-2 | $C_{18}H_{30}O_3$ | 294 |

(continued)

| Compound | Molecular formula | Molecular weight |
|---|---|---|
| R2-1 | $C_{18}H_{30}O_3$ | 294 |
| R2-2 | $C_{18}H_{30}O_3$ | 294 |
| R3 | $C_{18}H_{30}O_3$ | 294 |

As a result of analysis using NMR, it was found that the compound R1-2 had the following structure.

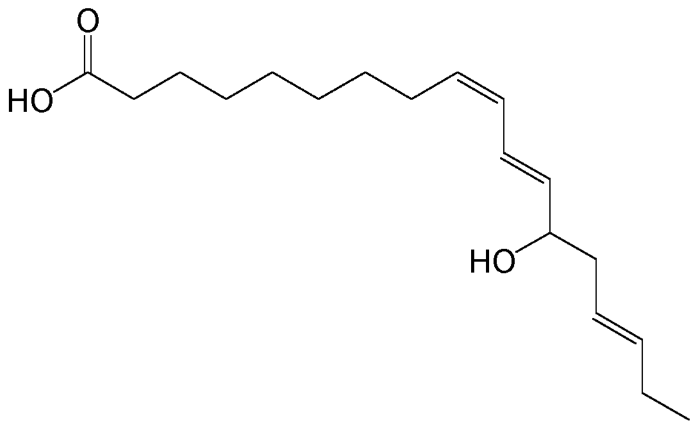

1H NMR spectra (500 MHz, CHLOROFORM-D) δ 0.96 (t, J = 7.7 Hz), 1.37 (q, J = 6.5 Hz), 1.60-1.66 (m), 2.04-2.09 (m), 2.15-2.19 (m), 2.28-2.38 (m), 4.23 (q, J = 6.3 Hz), 5.35 (q, J = 9.5 Hz), 5.41-5.46 (m), 5.54-5.59 (m), 5.68 (dd, J = 15.2, 6.6 Hz), 5.97 (t, J = 11.2 Hz), 6.52 (dd, J = 15.8, 11.7 Hz)
13C NMR spectra (126 MHz, CHLOROFORM-D) δ 14.3, 20.8, 24.7, 27.6, 28.9, 29.4, 33.8, 35.3, 72.2, 123.8, 126.0, 127.9, 133.0, 134.9, 135.4, 178.1

As a result of analysis using NMR, it was found that the compound R2-2 had the following structure.

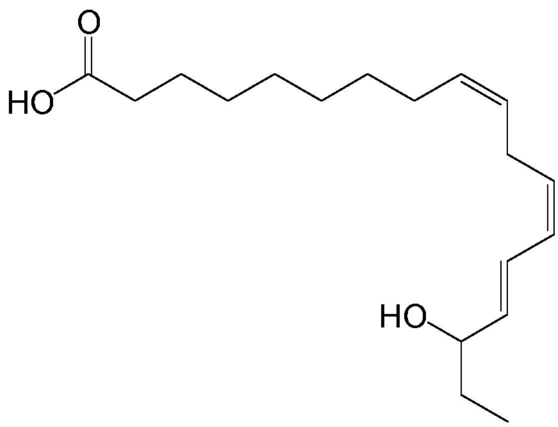

1H NMR spectra (500 MHz, CHLOROFORM-D) δ 6.52 (dd, J = 14.9, 10.9 Hz), 5.99 (t, J = 10.9 Hz), 5.68 (dd, J = 15.2, 6.6 Hz), 5.40 (q, J = 8.0 Hz), 5.39-5.31 (m), 4.12 (q, J = 6.5 Hz), 2.92 (t, J = 7.2 Hz), 2.34 (t, J = 7.4 Hz), 2.05 (q, J = 6.9 Hz), 1.66-1.59 (m), 1.60-1.55 (m), 1.25-1.39 (m), 0.93 (t, J = 7.4 Hz, 3H)
13C NMR spectra (126 MHz, CHLOROFORM-D) δ 178.4, 135.9, 130.8, 127.9, 127.3, 125.8, 77.4, 77.1, 76.9, 74.3, 33.8, 30.3, 29.5, 29.1, 29.0, 27.2, 26.2, 24.7, 9.8

As a result of analysis using NMR, it was found that the compound R3 had the following structure.

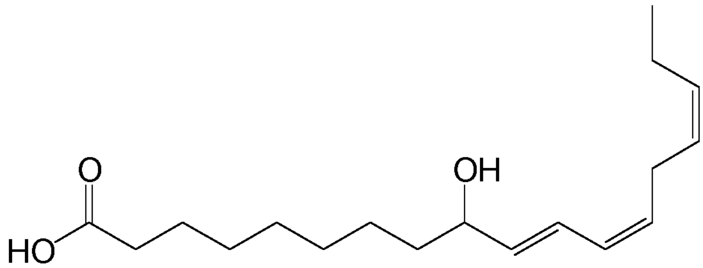

1H NMR spectra (500 MHz, CHLOROFORM-D) δ 6.50 (dd, J = 14.9, 10.9 Hz), 5.98 (t, J = 10.9 Hz), 5.68 (dd, J = 14.9, 6.9 Hz), 5.46-5.38 (m), 5.31 (q, J = 8.8 Hz), 4.16 (q, J = 6.5 Hz), 2.92 (t, J = 7.4 Hz), 2.34 (t, J = 7.7 Hz), 2.10-2.04 (m), 1.64-1.61 (m), 1.57-1.50 (m), 0.97 (t, J = 7.4 Hz)
13C NMR spectra (126 MHz, CHLOROFORM-D) δ 178.7, 136.3, 132.5, 130.9, 127.9, 126.6, 125.6, 73.0, 37.3, 33.9, 29.4, 29.2, 29.0, 26.1, 25.4, 24.7, 20.7, 14.3

2. TRPA1 activation test 1

(1) Preparation of expression vectors and stably expressing cell lines of TRPA1 and the like

DNAs encoding genes described in the following Table 2 were inserted into pcDNA 5/TO vector (Invitrogen) to prepare various expression vectors.

Table 2: Genes used and accession numbers of GenBank

| Gene | Accession number |
|---|---|
| Human TRPA1 (hTRPA1) | NM_007332.3 |
| Human TRPV1 (hTRPV1) | NM_080704.4 |
| Human TRPM8 (hTRPM8) | NM_024080.5 |

These expression vectors were transfected into T-REx™-293 cells (Invitrogen) by a conventional method using Lipofectamine LTX (Invitrogen). Then, various transfectants were cultured and cloned by a conventional method using Dulbecco's Modified Eagle Medium (DMEM, Nacalai Tesque Inc.), containing 10% fetal bovine serum (FBS, Cytiva), 400 μ/mL of hygromycin B, and 5 μg/mL of blasticidin S, as a drug selection medium to obtain a hTRPA1 stably expressing cell line, a hTRPV1 stably expressing cell line, and a hTRPM8 stably expressing cell line.

(2) Intracellular $Ca^{2+}$ imaging assay 1-1

A hTRPA1 stably expressing cell line in which expression of hTRPA1 had been induced in advance by a conventional method was prepared in a 96 well clear bottom plate (black), and a hTRPA1 activating action of the purified oxylipin or the R2 fraction (both were assumed to contain a compound having a molecular weight of 294) obtained in the above item 1 was tested. Specifically, after each well was washed with PBS, 50 μL of an assay buffer (Hanks' balanced salt solution containing no calcium ions or magnesium ions that contained 1 mM of $CaCl_2$ dihydrate, 20 mM of HEPES, and 0.1%BSA) containing calcium fluorescent indicator Calbryte™ 520 AM (produced by AAT Bioquest) in a final concentration of 4 μM was added, followed by incubating at 33°C for 45 minutes. After washing with the assay buffer, 100 μL of the assay buffer was added to each well. This plate was set to FlexStation 3 (Molecular Devices, LLC), and a fluorescence intensity (excitation wavelength 490 nm, fluorescence wavelength 525 nm) at 31°C was measured every two seconds, and 30 seconds after the start of the measurement, 100 μL of an assay buffer containing a test sample which was prepared to have a certain final concentration or DMSO was added. Then, a well in which an assay buffer containing ionomycin (having a final concentration of 10 μM after the addition) instead of the test samples was added was also prepared as a reference for fluorescence intensity.

The measurement was continued from the start of the measurement up to 120 seconds, and the TRPA1 activation ability of the test samples was calculated in accordance with the following formula. Results are shown in Table 3.

$$\text{Activation rate (\%)} = (F_{max[s]} - F_0)/(F_{max[I]} - F_0) \times 100$$

$F_{max[s]}$: The maximum value of the fluorescence intensity when the test sample or the positive control was added
$F_0$: The average value of the fluorescence intensity for 20 seconds after the start of the measurement (base line)
$F_{max[I]}$: The maximum value of the fluorescence intensity when ionomycin was added

Table 3: TRPA1 activation rate (average±standard error, n=5 or 8)

| Test sample | Activation rate |
|---|---|
| Purified oxylipin | |
| 500 μM | 93.4±5.62 |
| 250 μM | 56.6±2.14 |
| 100 μM | 35.6±0.883 |
| R2 fraction | |
| 500 μM | 105±4.41 |
| 250 μM | 45.5±2.66 |
| 100 μM | 34.1±1.98 |
| DMSO | 1.10±0.116 |

TRPA1 was able to be activated by using both of the purified oxylipin and the R2 fraction. While the purified oxylipin contained not only the R2 fraction but also the R1 fraction and R3 fraction, an activation rate comparable to that of the R2 fraction was obtained in the purified oxylipin even when such purified oxylipin was used with the same molar concentration as that of the R2 fraction alone (that is, even when the molar concentration of the R2 fraction contained in the purified oxylipin was lower than that of the R2 fraction alone). Hence, it is considered that not only the compound contained in the R2 fraction but also the compound contained in the R1 fraction or R3 fraction has a TRPA1 activating action.

(3) Intracellular $Ca^{2+}$ imaging assay 1-2

The TRPA1 activation ability was measured in the same manner as in the above (2) except that the R2 fraction or allyl isothiocyanate (AITC) was used as a test sample and the test was conducted with A-967079 (1 μM), which is a TRPA1-specific antagonist, or without A-967079. In addition, regarding the R2 fraction, the hTRPV1 stably expressing cell line or the hTRPM8 stably expressing cell line was used instead of the hTRPA1 stably expressing cell line to conduct the same test. Note that as positive controls, capsaicin was used for the hTRPV1 stably expressing cell line and icilin was used for the hTRPM8 stably expressing cell line. Results are shown in Table 4 and Table 5.

Table 4: TRPA1 activation rate (average±standard error, n=5)

| Test sample | Without antagonist | With antagonist |
|---|---|---|
| AITC 30 μM | 160±2.58 | 1.30±0.639 |
| R2 fraction | | |
| 500 μM | 88.9±0.972 | 3.33±0.792 |
| 100 μM | 43.9±1.59 | 0.734±0.0360 |
| DMSO | 0.920±0.177 | 0.612±0.116 |

Table 5: TRPV1 or TRPM8 activation rate (average±standard error, n=6 or 5)

| Test sample | TRPV1 | TRPM8 |
|---|---|---|
| Capsaicin 1 μM | 113±1.16 | - |
| Icilin 5 μM | - | 79.8±1.71 |
| R2 fraction | | |
| 500 μM | 2.08±0.183 | 3.76±0.258 |
| 100 μM | 0.542±0.0371 | 1.61±0.0640 |

(continued)

| | | |
|---|---|---|
| R2 fraction | | |
| DMSO | 0.239±0.0144 | 0.771±0.110 |

The R2 fraction exhibited the TRPA1 activating action as in the test result of the above (2), and this action was inhibited by the TRPA1-specific antagonist. In addition, by the R2 fraction, TRPV1 and TRPM8, which are other receptors belonging to the super family of the TRP ion channels, were not activated. Hence, it is considered that the compound contained in the R2 fraction has an action of specifically activating TRPA1.

(4) Intracellular $Ca^{2+}$ imaging assay 1-3

The TRPA1 activation ability was measured in the same manner as in the above (2) except that each isolated compound or allyl isothiocyanate (AITC) was used as a test sample and the test was conducted with A-967079 (1 μM), which was a TRPA1-specific antagonist, or without A-967079. Results are shown in Table 6.

Table 6: TRPA1 activation rate (average±standard error, n=5)

| Test sample | Without antagonist | With antagonist |
|---|---|---|
| AITC 30 μM | 128±2.99 | 1.44±0.163 |
| R1-2 | | |
| 500 μM | 95.9±1.79 | 8.24±1.13 |
| 200 μM | 32.0±1.40 | 1.75±0.443 |
| R2-1 | | |
| 500 μM | 104±2.03 | 4.02±0.0.186 |
| 200 μM | 32.5±3.26 | 1.20±0.276 |
| R2-2 | | |
| 500 μM | 77.5±0.988 | 1.96±0.548 |
| 200 μM | 21.6±5.63 | 0.71±0.0743 |
| R3 | | |
| 500 μM | 93.6±1.92 | 0.731±0.237 |
| 200 μM | 16.3±0.812 | 0.58±0.0564 |
| DMSO | 1.45±0.153 | 1.67±0.148 |

The compound R1-2, the compound R2-1, the compound R2-2, and the compound R3 exhibited the TRPA1 activating action, and this action was inhibited by the TRPA1-specific antagonist. Since all of the compound R1-2, the compound R2-1, the compound R2-2, and the compound R3 have a common chemical structure of an oxidized unsaturated fatty acid, it is considered that an oxylipin having such a chemical structure exhibits the TRPA1 activating action.

3. Anorexia improving test 1

Male ICR mice were housed in individual cages in which ALPHA-dri (Shepherd Specialty Papers) was laid, and were acclimatized and raised for at least one week or more while a powder feed (CE-2, CLEA Japan, Inc.) was given to the mice by using a powder feeder for mice (SN-950, Shinano Manufacturing Co., Ltd.). Then, for several days before the experiment, handling and oral administration training using a feeding needle (FG4202, Fuchigami Kikai Co., Ltd.) was conducted every day.

To 9 week-old mice under ad libitum feeding conditions, an oxylipin solution (2% by mass of purified oxylipin/ethanol, 10% Tween 80, and 88% saline) or a carrier solution was orally administered from 9:20 a.m. (10 mL/kg body weight). The dosage of the oxylipin was 757 μmol/kg body weight in terms of molar concentration. Then, a feeder the mass of which had been measured in advance was set in the cage at 9:30 a.m. The masses of the feeder and the feed spilled in the cage 0.5, 1, 2, 3, and 6 hours after the start of the feeding experiment were measured, and the ingested mass (g) at each time was measured. Results are shown in Fig. 2.

The cumulative amounts of food ingested of the mice were increased by the administration of the oxylipin solution (Fig. 2). This is considered to be because the appetites of the mice were increased by the administration of the

oxylipin solution.

4. TRPA1 activation test 2

(1) Intracellular $Ca^{2+}$ imaging assay 2-1

A hTRPA1 stably expressing cell line in which expression of hTRPA1 had been induced in advance by a conventional method was prepared in a 96 well clear bottom plate, and the hTRPA1 activating action of rhein (produced by Tokyo Chemical Industry Co., Ltd.) was tested. Specifically, after each well was washed with PBS, 50 μL of an assay buffer (Hanks' balanced salt solution containing no calcium ion or magnesium ion that contained 1 mM of $CaCl_2$ dihydrate, 20 mM of HEPES, and 0.1%BSA) containing calcium fluorescent indicator Calbryte™ 520 AM (produced by AAT Bioquest) in a final concentration of 4 μM was added, followed by incubating at 33°C for 45 minutes. After washing with the assay buffer, 100 μL of the assay buffer was added to each well. This plate was set to FlexStation 3 (Molecular Devices, LLC), and a fluorescence intensity (excitation wavelength 490 nm, fluorescence wavelength 525 nm) at 31°C was measured every two seconds, and 30 seconds after the start of the measurement, 100 μL of an assay buffer containing a test sample (allyl isothiocyanate (AITC) of positive control or rhein) which was prepared to have a certain final concentration or DMSO was added. Then, a well in which an assay buffer containing ionomycin (having a final concentration of 10 μM after the addition) instead of the test samples was added was also prepared as a reference for fluorescence intensity.

The measurement was continued from the start of the measurement up to 120 seconds, and the TRPA1 activation ability of the test samples was calculated in accordance with the following formula. Results are shown in Table 7.

$$\text{Activation rate (\%)} = (F_{max[s]} - F_0)/(F_{max[I]} - F_0) \times 100$$

$F_{max[s]}$: The maximum value of the fluorescence intensity when the test sample or positive control was added
$F_0$: The average value of the fluorescence intensity for 20 seconds after the start of the measurement (base line)
$F_{max[I]}$: The maximum value of the fluorescence intensity when ionomycin was added

Table 7: TRPA1 activation rate (average±standard error, n=8)

| Test sample | | Activation rate |
|---|---|---|
| AITC 30 μM | | 101±3.66 |
| Rhein | | |
| | 200 μM | 85.7±1.62 |
| | 100 μM | 75.5±1.19 |
| | 75 μM | 69.7±1.25 |
| | 50 μM | 66.4±1.45 |
| | 25 μM | 41.1±1.51 |
| | 10 μM | 19.1±1.00 |
| | 1 μM | 0.57±0.0414 |

Rhein activated TRPA1 in concentration dependent manner. Hence, it was indicated that rhein had a TRPA1 activating action.

(2) Intracellular $Ca^{2+}$ imaging assay 2-2

The TRPA1 activation ability of each test sample was measured in the same manner as in the above (2) except the test was conducted with A-967079 (1 μM), which is a TRPA1-specific antagonist, or without A-967079. In addition, intracellular $Ca^{2+}$ imaging assay was conducted in the same manner as in the above (2) except that a hTRPV1 stably expressing cell line, a hTRPM8 stably expressing cell line, or a Mock cell was used instead of the hTRPA1 stably expressing cell line. Note that capsaicin was used as positive control for the hTRPV1 stably expressing cell line, and icilin was used as positive control for the hTRPM8 stably expressing cell line. Results are shown in Table 8 and Table 9.

Table 8: TRPA1 activation rate (average±standard error, n=6)

| Test sample | Without antagonist | With antagonist |
|---|---|---|
| AITC 30 μM | 104±6.10 | 15.7±7.81* |
| Rhein | | |
| 200 μM | 82.1±0.918 | 8.40±0.238* |
| 100 μM | 79.4±4.08 | 3.26±0.102* |
| *p<0.05 (A multiple testing in the Tukey's method, the significant difference from the test plot without an antagonist) | | |

Table 9: TRPV1 or TRPM8 activation rate (average±standard error, n=6 to 9)

| Test sample | TRPV1 | TRPM8 | Mock |
|---|---|---|---|
| Capsaicin 1 μM | 96.3±3.24 | - | - |
| Icilin 5 μM | - | 104±2.19 | - |
| AITC 30 μM | - | - | 6.40±0.143 |
| Rhein | | | |
| 200 μM | 5.49±0.0830 | 5.71±0.118 | 9.65±0.143 |
| 100 μM | 2.40±0.0395 | 2.52±0.0847 | 3.49±0.0595 |

The TRPA1 activating action of rhein was inhibited by the TRPA1-specific antagonist. In addition, TRPV1 and TRPM8, which are other receptors belonging to the super family of the TRP ion channels, were not activated by rhein. Hence, it is considered that rhein has an action of specifically activating TRPA1.

(3) Intracellular $Ca^{2+}$ imaging assay 2-3

A TRPA1 activation ability was measured in the same manner as in the above (2) by using, as a test sample, rhein or compounds described in the following Table 10, which had anthraquinone structures similar to rhein, at a concentration of 200 μM of the test sample. Results are shown in Table 10.

Table 10: Structures and TRPA1 activation rates (average±standard error, n=6 to 8) of test samples

| | |
|---|---|
| Rhein<br>85.7±1.62<br>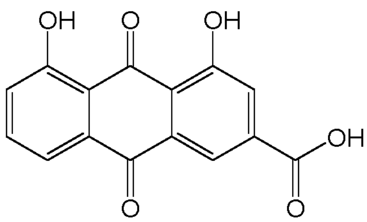 | Emodin<br>43.1±1.72<br>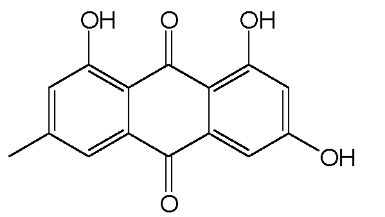 |
| Physcion<br>24.3±0.760<br>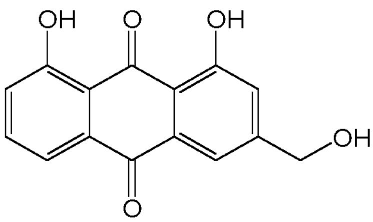 | Aloe-emodin<br>15.3±0.743<br>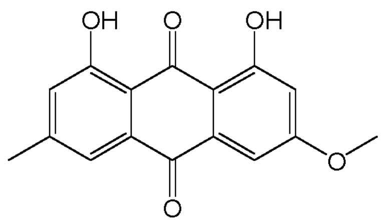 |
| Dantron<br>4.72±0.762 | Chrysophanol<br>3.27±0.439 |

(continued)

| | |
|---|---|
| 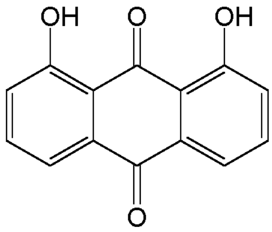 | 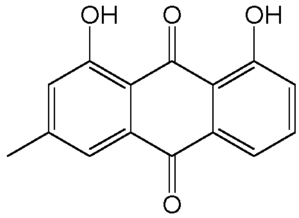 |
| Sennoside A<br>3.33±0.705<br>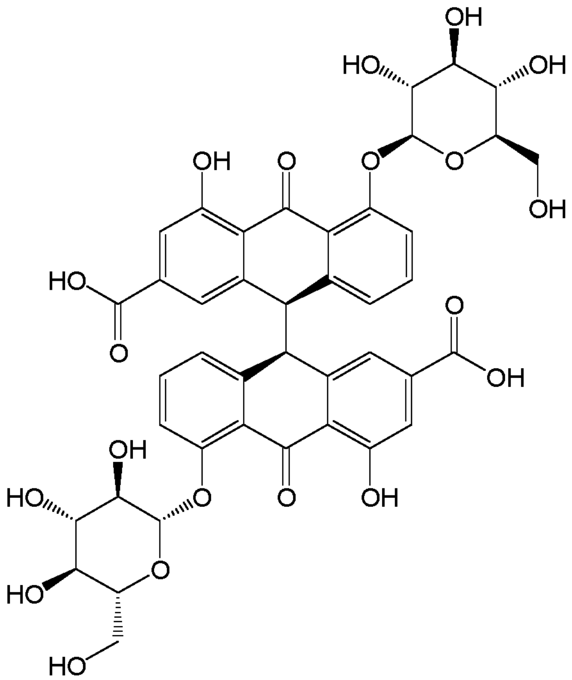 | Anthrarufin<br>0.587±0.0172<br>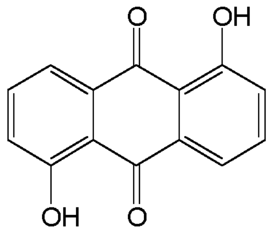 |

Among the compounds having anthraquinone structures, emodin, aloe-emodin, and physcion exhibited TRPA1 activating actions. It was also confirmed that these compounds exhibited TRPA1 activity in concentration dependent manner from additional experiments. In addition, as shown in the following Table 11, the TRPA1 activating action of emodin was inhibited by A-967079, which is a TRPA1-specific antagonist.

Table 11: TRPA1 activation rate (average±standard error, n=6)

| Test sample | Without antagonist | With antagonist |
|---|---|---|
| AITC 30 μM | 115±6.15 | 0.811±0.0386* |
| Emodin | | |
| 400 μM | 71.29±1.08 | 6.25±1.70* |
| 200 μM | 64.8±3.73 | 2.96±0.246* |
| 100 μM | 49.4±1.41 | 0.881±0.134* |
| *p<0.05 (A multiple testing in the Tukey's method, the significant difference from the test plot without an antagonist) | | |

Since all of rhein, emodin, aloe-emodin, and physcion have a 1,8-dihydroxyanthraquinone structure including a substituent containing an oxygen atom at the 3 position, it was suggested that this structure was necessary for activating TRPA1.

5. Anorexia improving test 2

Male ICR mice were housed in individual cages in which ALPHA-dri (Shepherd Specialty Papers) was laid, and were acclimatized and raised for at least one week or more while a powder feed (CE-2, CLEA Japan, Inc.) was given to the mice by using a powder feeder for mice (SN-950, Shinano Manufacturing Co., Ltd.). Then, for several days before the experiment, handling and oral administration training using a feeding needle (FG4202, Fuchigami Kikai Co., Ltd.) were conducted every day.

To 9 week-old mice under ad libitum feeding conditions, a carrier solution (containing 2% of dimethyl sulfoxide and 98% of a 0.5% methyl cellulose solution in volume ratio) or a rhein solution (obtained by dissolving 8.53 mg of rhein in 1 mL of the carrier solution) was orally administered from 9:20 a.m. (10 mL/kg body weight). The dosage of rhein was 300 μmol/kg body weight in terms of molar concentration. Then, a feeder the mass of which had been measured in advance was set in the cage at 9:30 a.m. The masses of the feeder and the feed spilled in the cage 0.5, 1, 2, and 3 hours after the start of the feeding experiment were measured, and the ingested mass (g) at each time was measured (n=6). Results are shown in Fig. 3.

The cumulative amounts of food ingested of the mice were increased by the administration of the rhein solution (Fig. 3). This is considered to be because the appetites of the mice were increased by the administration of the rhein solution.

In addition, when rhein was administered to mice in 100 μmol/kg body weight as well, the cumulative amounts of food ingested were significantly increased (Fig. 4, n=5).

6. Anorexia improving test 3

The ingested mass (g) of each mouse was measured in the same manner as in anorexia improving test 2 (the above item 5) except that 10 mL/kg body weight of a carrier solution, a rhein solution (100 μmol/kg body weight), an A-967079 solution (10 μmol/kg body weight), or a mixed solution of A-967079 and rhein (in 10 μmol/kg body weight and 100 μmol/kg body weight, respectively) was orally administered to the mouse. Results obtained by calculating the cumulative amounts of food ingested and conducting a multiple comparison (multiple t-test) by the Dunn-Bonferroni method are shown in Table 12.

Table 12: Cumulative amounts of food ingested of mice (average±standard error, n=5 or 6)

| Time after the administration (hour) | 0.5 | 1 | 2 | 3 |
|---|---|---|---|---|
| Carrier solution | 0.030±0.024 | 0.045±0.023 | 0.145±0.039 | 0.24±0.018 |
| Rhein | 0.21±0.033* | 0.25±0.027* | 0.42±0.020* | 0.49±0.035* |
| A-967079 | 0.026±0.0093 | 0.042±0.015 | 0.088±0.027 | 0.20±0.066 |
| A-967079+rhein | 0.12±0.059 | 0.15±0.051 | 0.19±0.080 | 0.28±0.079 |

*p<0.05 (multiple t-test, the significant difference from the corresponding test plot without rhein)

The significant increase in the cumulative amounts of food ingested observed by the administration of rhein (the upper stage in Table 12) was not observed in the case where A-967079, which is a TRPA1-specific antagonist, was administered (the lower stage in Table 12). Hence, it was proven that the action of increasing the amount of food ingested by an active ingredient having the TRPA1 activating action such as rhein was achieved via the activation of TRPA1.

As described above, it was found that an oxidized unsaturated fatty acid such as an oxylipin having a TRPA1 activating action can stimulate appetite to increase the amount of food ingested. In addition, it was found that a compound represented by the formula (1), such as rhein, has a TRPA1 activating action, and this compound can stimulate the appetite to increase the amount of food ingested. Therefore, it becomes possible to improve anorexia by using an oxidized unsaturated fatty acid such as oxylipins or a compound represented by the formula (1) such as rhein that has a TRPA1 activating action.

## Claims

**1.** A composition for increasing an amount of food ingested and/or improving anorexia, the composition comprising

(A) a fatty acid having 12 to 26 carbon atoms, 2 or more carbon-carbon double bonds, and 1 to 3 substituents selected from the group consisting of a hydroxyl group and a hydroperoxide group, or a pharmaceutically

acceptable salt thereof, or

a fatty acid which is an oxylipin having a transient receptor potential ankyrin 1 (TRPA1) activating action, or a pharmaceutically acceptable salt thereof, or

(B) a compound represented by formula (1):

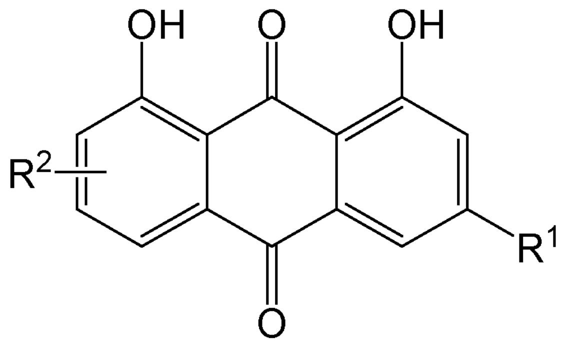

(1)

wherein

$R^1$ is -COOH, -OH, $-CH_2$-OH, or -O-$CH_3$, and

$R^2$ is -H, $-CH_3$, or -OH, or a glycoside thereof, or a pharmaceutically acceptable salt thereof.

**2.** The composition according to claim 1, wherein the fatty acid is represented by formula (1'):

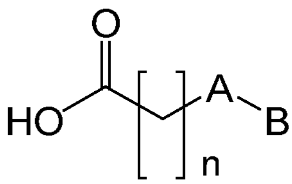

(1')

wherein

n is an integer of 3 to 9,

A is a saturated or unsaturated linear hydrocarbon group having 3 to 9 carbon atoms,

B is an unsaturated linear hydrocarbon group having 5 to 7 carbon atoms, and

A and B have a total of 2 or more carbon-carbon double bonds and a total of 1 to 3 substituents selected from the group consisting of a hydroxyl group and a hydroperoxide group.

**3.** The composition according to claim 2, wherein in the formula (1'), n is an integer of 6 to 8 and/or A is an unsaturated linear hydrocarbon group having 5 to 7 carbon atoms.

**4.** The composition according to claim 2, wherein in the formula (1'), B is

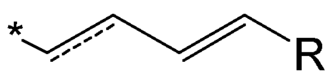

or

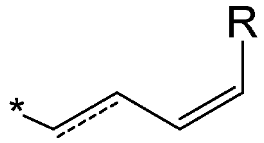

wherein asterisk represents a binding site with A, a solid double line and a dashed double line represent a single bond or a double bond, and R is an alkyl group having 1 to 3 carbon atoms.

5. The composition according to claim 2, wherein in the formula (1'), A and B have a total of 2 to 5 carbon-carbon double bonds.

6. The composition according to claim 2, wherein in the formula (1'),

   n is an integer of 6 to 8,
   A is an unsaturated linear hydrocarbon group having 5 to 7 carbon atoms, and
   A and B have a total of 3 carbon-carbon double bonds and a total of one hydroxyl or hydroperoxide group.

7. The composition according to claim 1, wherein a molecular formula of the fatty acid is $C_{18}H_{30}O_3$ or $C_{18}H_{32}O_3$.

8. The composition according to claim 1, wherein the fatty acid has a structure represented by any of the following chemical formulas:

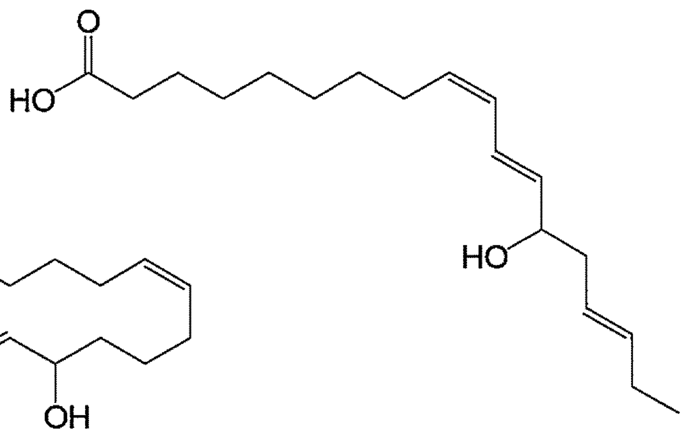

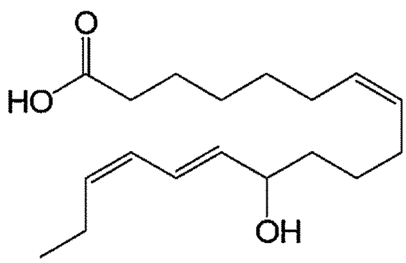

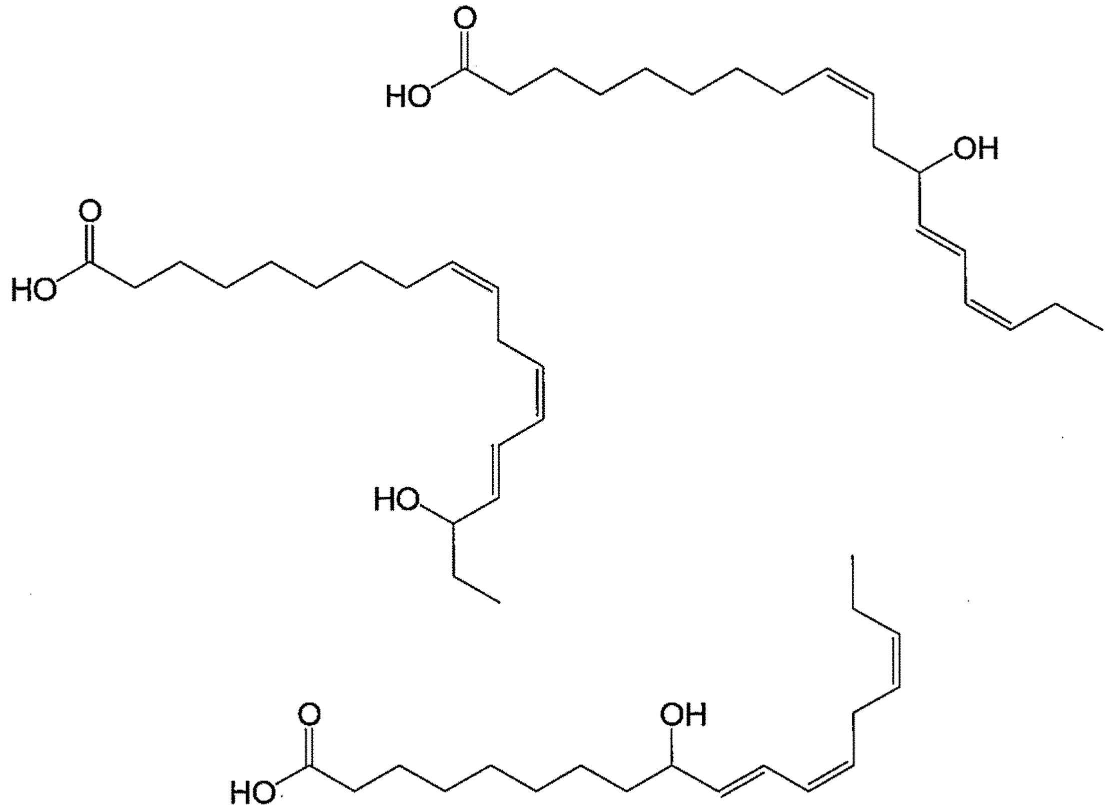

9. The composition according to claim 1, wherein the compound represented by the formula (1) is represented by formula (2):

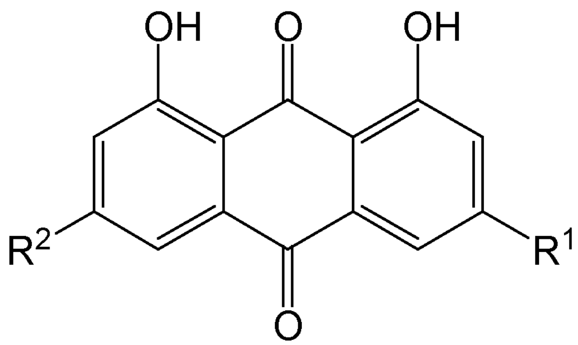

(2)

wherein

$R^1$ is -COOH, -OH, $-CH_2-OH$, or $-O-CH_3$, and
$R^2$ is -H, $-CH_3$, or -OH.

10. The composition according to claim 1, wherein the compound represented by the formula (1) has any of the following structures:

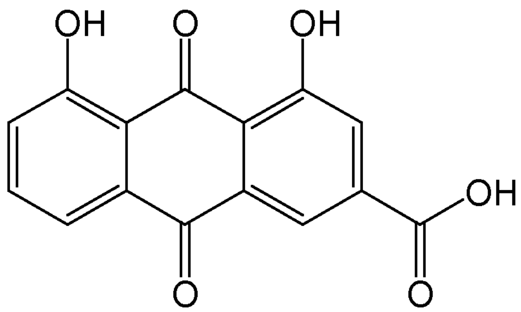

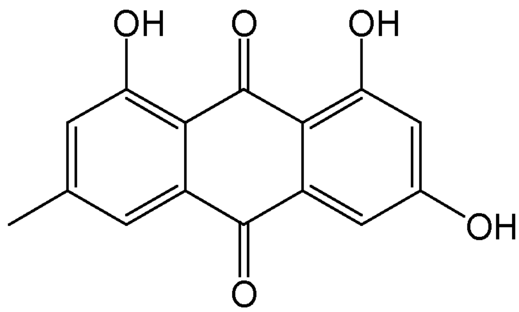

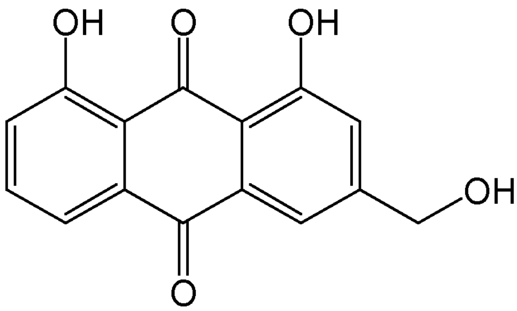

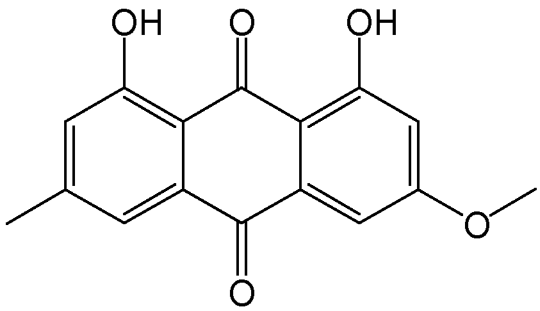

**11.** The composition according to any one of claims 1 to 10, that is a food composition or a pharmaceutical composition.

**12.** A composition for activating transient receptor potential ankyrin 1 (TRPA1), comprising a compound represented by formula (1):

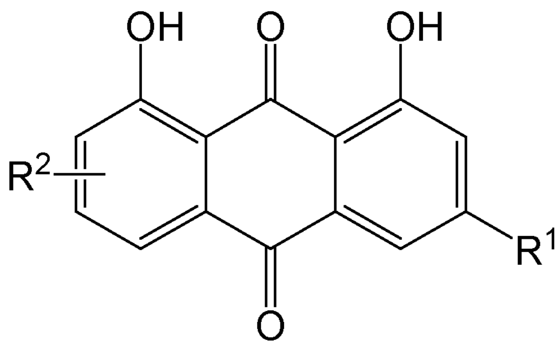

(1)

wherein

$R^1$ is -COOH, -OH, $-CH_2$-OH, or -O-$CH_3$, and
$R^2$ is -H, $-CH_3$, or -OH, or a glycoside thereof, or a pharmaceutically acceptable salt thereof.

**13.** The composition according to claim 12, wherein the compound represented by the formula (1) is represented by formula (2):

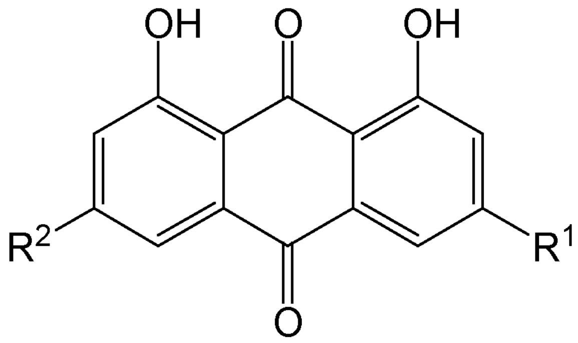

(2)

wherein

$R^1$ is -COOH, -OH, $-CH_2-OH$, or $-O-CH_3$, and
$R^2$ is -H, $-CH_3$, or -OH.

**14.** The composition according to claim 12, wherein the compound represented by the formula (1) has any of the following structures:

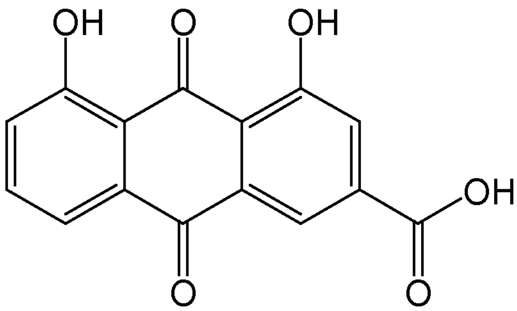

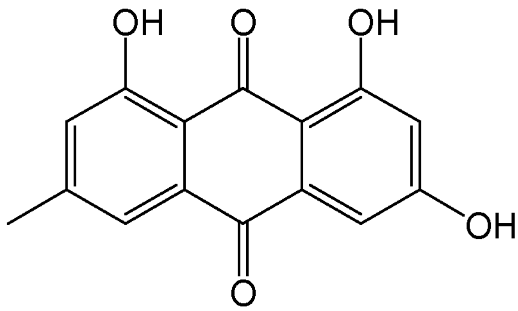

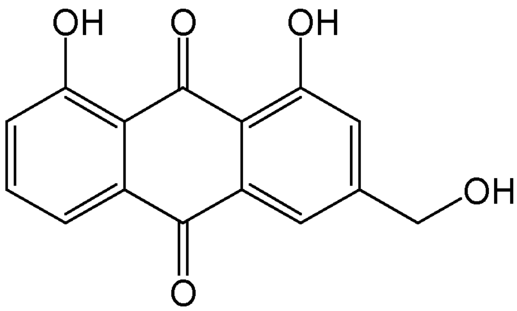

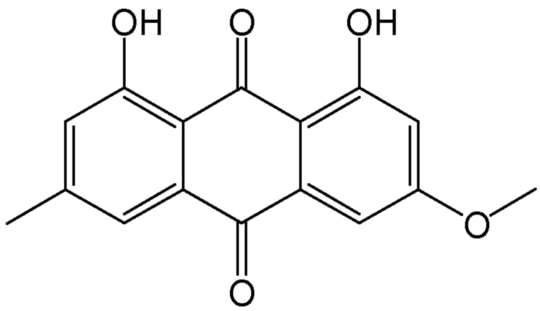

**15.** The composition according to any one of claims 12 to 14, that is a food composition, a pharmaceutical composition, or a pesticide composition.

FIG.1A

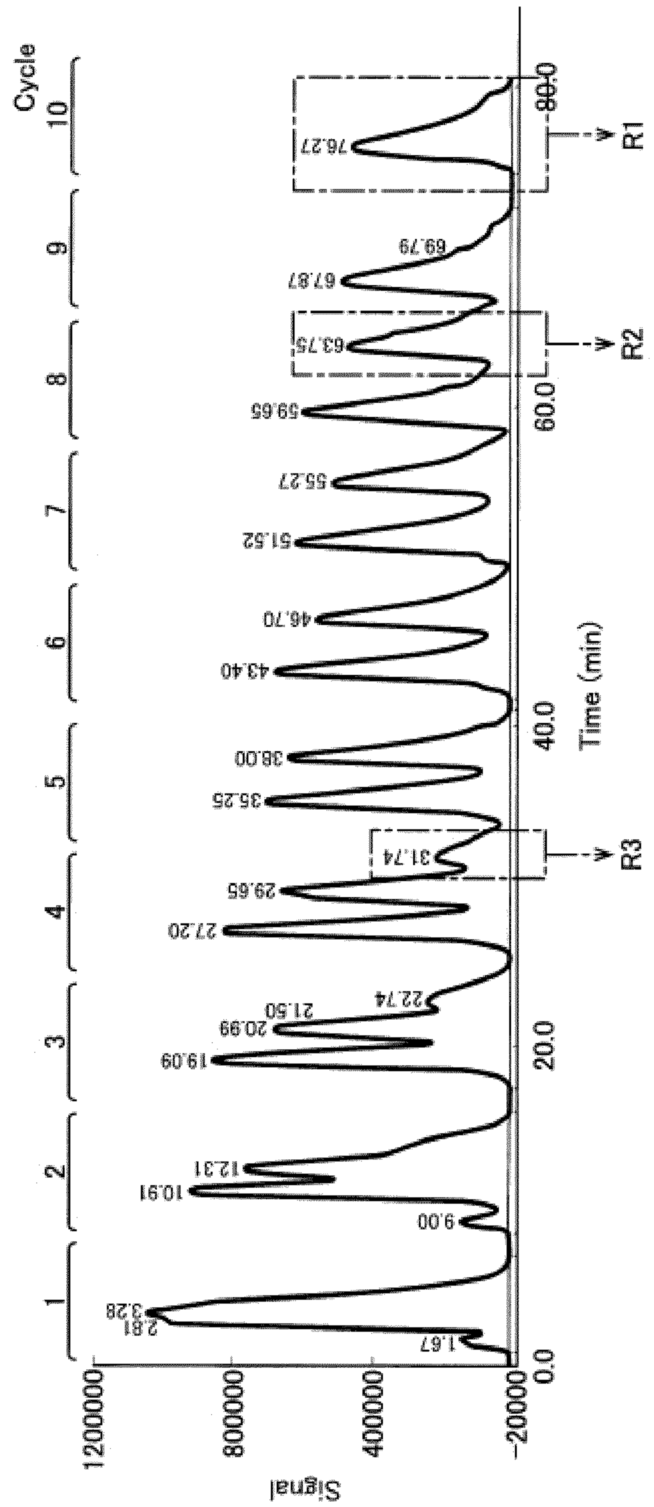
Cycle
1
2
3
4
5
6
7
8
9
10
Signal
12000000
8000000
4000000
−20000
0.0
20.0
40.0
60.0
80.0
Time (min)
1.67
2.81
3.28
9.00
10.91
12.31
19.09
20.99
21.50
22.74
27.20
29.65
31.74
35.25
38.00
43.40
46.70
51.52
55.27
59.65
63.75
67.87
69.79
76.27
R1
R2
R3

FIG.1B

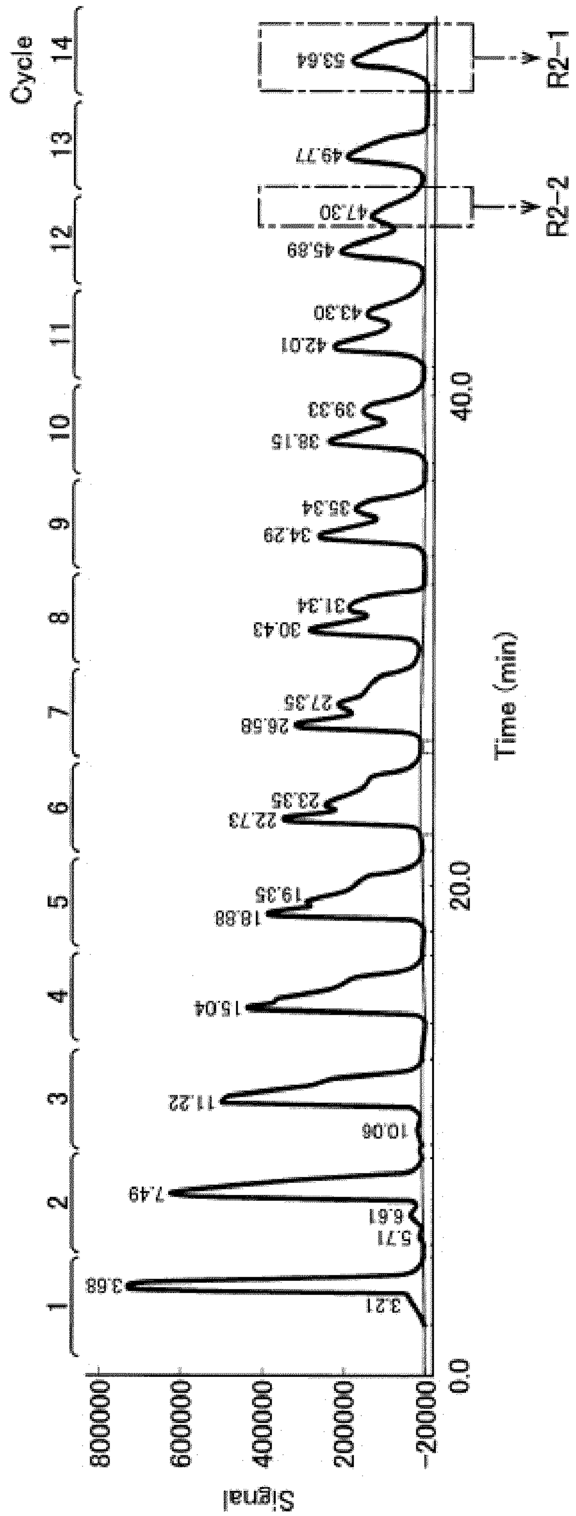
Cycle
1
2
3
4
5
6
7
8
9
10
11
12
13
14
3.21
3.68
5.71
6.61
7.49
10.06
11.22
15.04
18.88
19.35
22.73
23.35
26.58
27.35
30.43
31.34
34.29
35.34
38.15
39.33
42.01
43.30
45.89
47.30
49.77
53.64
R2-1
R2-2
Signal
800000
600000
400000
200000
-20000
0.0
20.0
40.0
Time (min)

FIG.1C

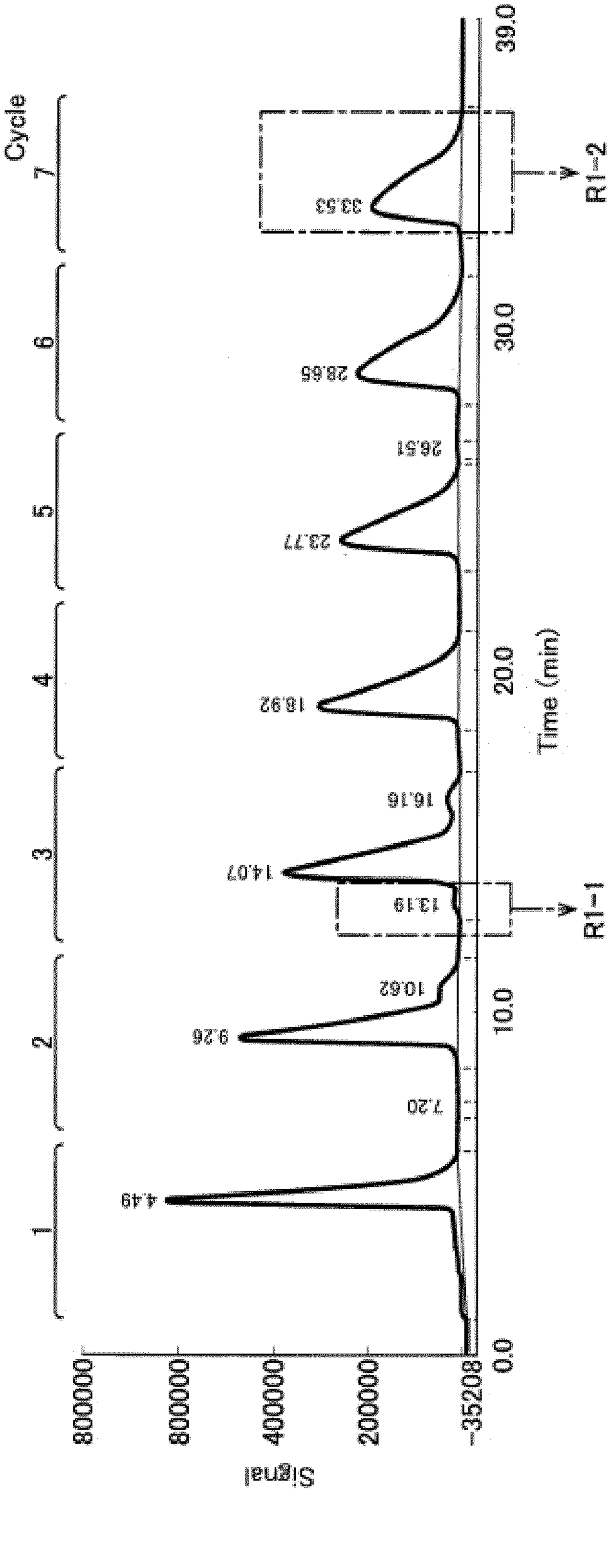
Cycle
1
2
3
4
5
6
7
4.49
7.20
9.26
10.62
13.19
14.07
16.16
18.92
23.77
26.51
28.65
33.53
R1-1
R1-2
Signal
800000
800000
400000
200000
-35208
0.0
10.0
20.0
30.0
39.0
Time (min)

FIG.2

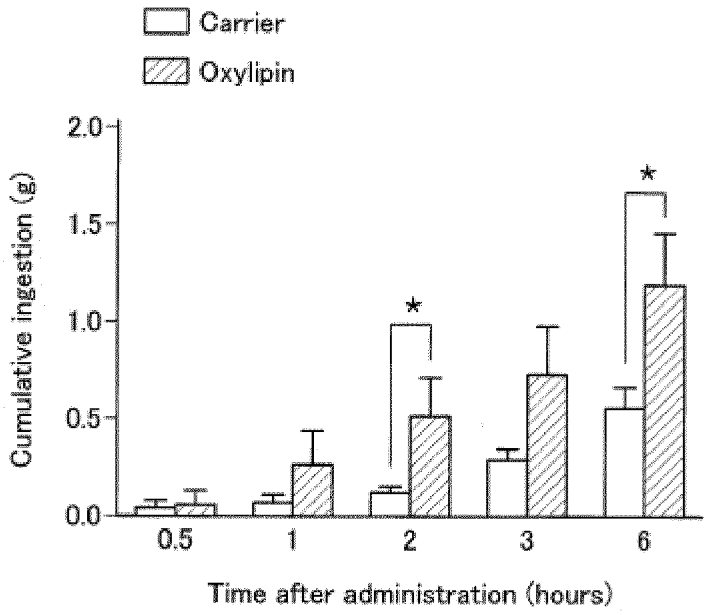
Carrier
Oxylipin
Cumulative ingestion (g)
2.0
1.5
1.0
0.5
0.0
*
*
0.5
1
2
3
6
Time after administration (hours)

FIG.3

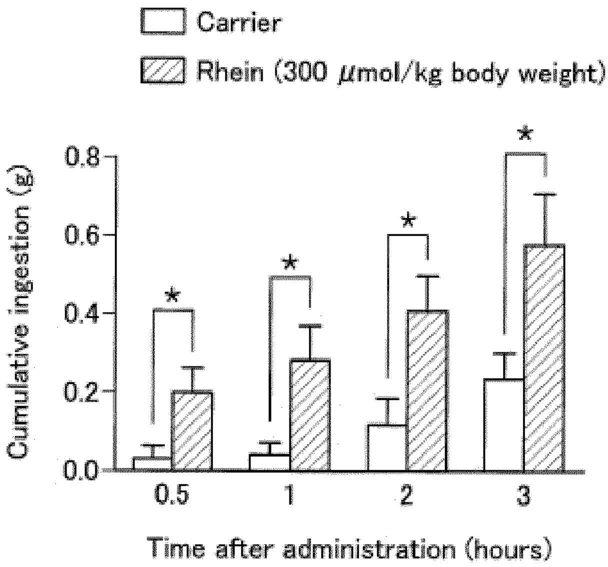
Carrier
Rhein (300 μmol/kg body weight)
Cumulative ingestion (g)
0.8
0.6
0.4
0.2
0.0
*
*
*
*
0.5
1
2
3
Time after administration (hours)

FIG.4

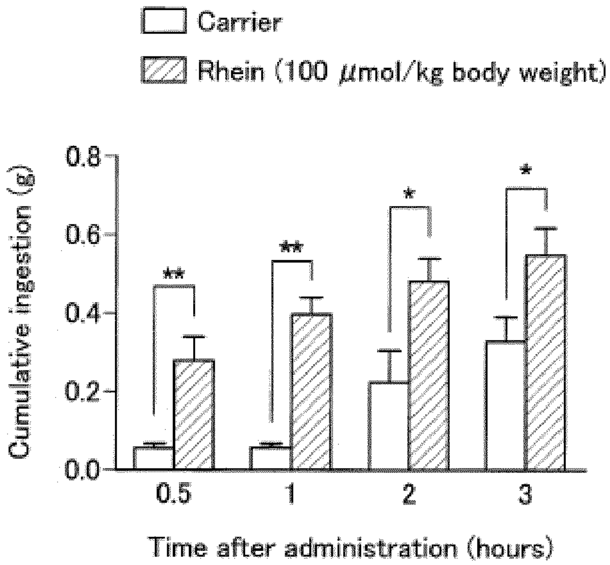
Carrier
Rhein (100 μmol/kg body weight)
Cumulative ingestion (g)
0.8
0.6
0.4
0.2
0.0
**
**
*
*
0.5
1
2
3
Time after administration (hours)

## INTERNATIONAL SEARCH REPORT

International application No.
**PCT/JP2022/042367**

**A. CLASSIFICATION OF SUBJECT MATTER**

***A23L 33/10***(2016.01)i; ***A23L 33/105***(2016.01)i; ***A23L 33/12***(2016.01)i; ***A61K 31/122***(2006.01)i; ***A61K 31/192***(2006.01)i; ***A61K 31/201***(2006.01)i; ***A61P 1/14***(2006.01)i; ***A61P 25/02***(2006.01)i; ***A61P 43/00***(2006.01)i; ***C07C 50/16***(2006.01)i; ***C07C 66/02***(2006.01)i

FI: A23L33/10; A61P25/02 101; A61P1/14; A61P43/00 111; A23L33/12; A61K31/192; A61P25/02; C07C66/02; C07C50/16; A61K31/201; A23L33/105; A61K31/122

According to International Patent Classification (IPC) or to both national classification and IPC

**B. FIELDS SEARCHED**

Minimum documentation searched (classification system followed by classification symbols)

A23L33/10; A23L33/105; A23L33/12; A61K31/122; A61K31/192; A61K31/201; A61P1/14; A61P25/02; A61P43/00; C07C50/16; C07C66/02

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2010-519311 A (MARTEK BIOSCIENCES CORPORATION) 03 June 2010 (2010-06-03) claims, examples, fig. 1-31, paragraphs [0072]-[0089], [0153] | 1-6 |
| A | | 7-15 |
| X | US 2007/0248586 A1 (MARTEK BIOSCIENCES CORPORATION) 25 October 2007 (2007-10-25) claims, examples, fig. 1-6, paragraphs [0071]-[0088], [0151] | 1-6 |
| A | | 7-15 |
| X | JP 2020-504118 A (TAIRX, INC.) 06 February 2020 (2020-02-06) claims, examples, paragraph [0004] | 1, 9-15 |
| A | | 2-8 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 December 2022** | **17 January 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/042367**

**C. DOCUMENTS CONSIDERED TO BE RELEVANT**

| Category* | Citation of document, with indication, where appropriate, of the relevant passages | Relevant to claim No. |
|---|---|---|
| X | JP 10-130201 A (EISAI CO LTD) 19 May 1998 (1998-05-19) claims, examples, paragraph [0011] | 1, 9, 11-13, 15 |
| A | | 2-8, 10, 14 |
| Y | JP 2014-76979 A (SAPPORO BREWERIES LTD) 01 May 2014 (2014-05-01) claims, examples | 1-8 |
| A | | 9-15 |
| Y | BANG, S. et al. Resolvin D1 attenuates activation of sensory transient receptor potential channels leading to multiple anti-nociception. British Journal of Pharmacology. 2010, vol. 161, no. 3, pp. 707-720 fig. 1-2, 7 | 1-8 |
| A | | 9-15 |
| Y | JP 2020-109065 A (UNIV JICHI MEDICAL) 16 July 2020 (2020-07-16) claims, examples | 1-8 |
| A | | 9-15 |
| A | WO 2017/081296 A1 (UCL BUSINESS PLC) 18 May 2017 (2017-05-18) claims | 1-15 |

Form PCT/ISA/210 (second sheet) (January 2015)

**INTERNATIONAL SEARCH REPORT**
**Information on patent family members**

International application No.
**PCT/JP2022/042367**

| Patent document cited in search report | Publication date (day/month/year) | Patent family member(s) | Publication date (day/month/year) |
|---|---|---|---|
| JP 2010-519311 A | 03 June 2010 | US 2011/0190389 A1<br>claims, examples, fig. 1-31, paragraphs [0116]-[0137], [0202]<br>WO 2008/103753 A2<br>EP 2120920 A2<br>CN 101663031 A | |
| US 2007/0248586 A1 | 25 October 2007 | JP 2008-520739 A<br>US 2006/0241088 A1<br>US 2011/0112191 A1<br>US 2009/0318394 A1<br>US 2011/0178047 A1<br>US 2009/0320148 A1<br>WO 2007/090162 A2<br>WO 2006/055965 A2<br>EP 1983977 A2<br>EP 1824809 A2<br>KR 10-2007-0090928 A<br>CN 101102988 A | |
| JP 2020-504118 A | 06 February 2020 | US 2018/0200217 A1<br>claims, examples<br>WO 2018/136266 A1<br>EP 3570825 A1<br>CN 110177547 A<br>KR 10-2019-0124713 A | |
| JP 10-130201 A | 19 May 1998 | (Family: none) | |
| JP 2014-76979 A | 01 May 2014 | (Family: none) | |
| JP 2020-109065 A | 16 July 2020 | (Family: none) | |
| WO 2017/081296 A1 | 18 May 2017 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018177739 A
- JP 2017518963 W
- JP 2017096785 A
- JP 2014210725 A
- JP 2014169240 A
- JP 2014076979 A
- JP 2014024810 A
- JP 2011506289 W
- WO 2009123080 A
- JP 2020109065 A
- JP 2015231953 A


### Non-patent literature cited in the description


- **MISHIMA KAIUN.** *Memorial Foundation Research Report,* 2014, vol. 51, 53-56